# EUROPÄISCHE PATENTANMELDUNG

(11) **EP 4 417 134 A1**
(43) Veröffentlichungstag der Anmeldung: **21.08.2024**
(21) Anmeldenummer: 24157941.6
(22) Anmeldetag: 15.02.2024
(51) Int. Cl.: A61B 7/04, A61B 5/0205, A61B 5/00, A61B 7/02

(54) **VORRICHTUNG UND VERFAHREN ZUR VERFASSUNG UND AUSWERTUNG FIBROAKUSTISCHER SIGNALE**

(30) Priorität: 16.02.2023 DE 102023103883
(71) Anmelder: SURAG Medical GmbH, 04103 Leipzig (DE)
(72) Erfinder: Illanes Manríquez, Alfredo Guillermo, 39108 Magdeburg (DE); Sühn, Thomas, 39108 Magdeburg (DE); Esmaeili, Nazila, 39108 Magdeburg (DE); Spiller, Moritz Herbert, 39104 Magdeburg (DE)
(74) Vertreter: Schneiders & Behrendt Bochum

(57) **Zusammenfassung**

Die Erfindung betrifft eine Vorrichtung zum Anschluss an ein medizinisches Instrument zur Erfassung von vibroakustischen Signalen, wobei die Vorrichtung eine Signalerfassungsvorrichtung (4) mit einem Kopplungselement (4a) zum Anschluss eines medizinischen Instruments (2), zumindest einen Sensor (4c) zur Erfassung vibroakustischer Signale, eine Analog-Digital-Wandlung (4e) zur Umwandlung der von dem mindestens einen Sensor erfassten analogen Signale in digitale Daten, einen Controller (4f) und eine Kommunikationseinheit (4g) zum Datenaustausch umfasst. Die Erfindung betrifft zudem ein computerimplementiertes Verfahren zur Auswertung der vibroakustischen Signale.

## Beschreibung

Die Erfindung betrifft eine Vorrichtung und ein Verfahren zur Erfassung und zur Auswertung vibroakustischer Signale insbesondere bei und im Zusammenhang mit minimalinvasiven Interventionen.

In der konventionellen offenen Chirurgie sind sensorische Eindrücke eine wichtige Informationsquelle für den Chirurgen. Der Chirurg verlässt sich während eines Eingriffs auf den visuellen, den taktilen und den auditiven Sinn, um den Zustand von Organen und Geweben bewerten zu können. Mit dem visuellen Sinn können Chirurgen beispielsweise das gesamte Operationsgebiet direkt überblicken und die betroffenen Körperregionen des Patienten beobachten; mit dem taktilen Sinn können sie Organe und Gewebe direkt mit der Hand untersuchen; und mit dem auditiven Sinn können Chirurgen die Geräusche von Organen wie Herz, Lunge und Gefäßen hören.

Es ist bekannt, dass diese sensorischen Eindrücke insbesondere bei minimalinvasiven Eingriffen (MIS) wenn überhaupt sowohl nur sehr reduziert als auch nur indirekt über ein medizinisches Instrument wahrnehmbar sind.

MIS ist zum Standard für viele chirurgische Eingriffe geworden. Gewöhnlich werden bei minimal invasiven Eingriffen spezielle medizinische Instrumente durch kleine Schnitte in den Körper eingeführt. Die Instrumente weisen entsprechend regelmäßig eine längliche Form mit einem möglichst geringen Durchmesser auf.

Die MIS hat für den Patienten enorme Vorteile gegenüber offen chirurgischen Eingriffen wie beispielsweise kürzere Erholungszeiten und ein geringeres Gewebetrauma, jedoch können die nur begrenzt verfügbaren sensorischen Informationen, die dem Chirurgen bei der MIS zur Verfügung stehen, zu einer höheren Anzahl von Fehlern und somit Komplikationen führen.

Die MIS stellt an die Chirurgen besondere Anforderungen auch im visuellen Bereich, da eine visuelle Übertragung in der Regel nur über Kameras erfolgt, wobei die Kameras ebenfalls minimalinvasiv eingeführt werden.

Auch ist die akustische Wahrnehmung der Organe im Gegensatz zur offenen Chirurgie eingeschränkt, da der Operationsbereich im Prinzip geschlossen ist und kein direkter Zugang zum Körperinneren des Patienten besteht.

Ferner wird der Tastsinn nahezu ausgeschaltet, da die Chirurgen ihre Finger nicht in den Körper des Patienten einführen können, sondern über mehr oder weniger starre Bedienelemente spezielle minimalinvasive Instrumente bedienen. Entsprechend können sich Chirurgen nicht auf ihre bei konventionellen Operationen erworbenen Fähigkeiten und Erfahrungen verlassen, um den Zustand des Patienten, der Organe oder Gewebe zu erfassen und zu bewerten.

Um den Chirurgen bei minimalinvasiven Eingriffen zu unterstützen und die fehlende Sensorik zumindest teilweise zu ersetzen, wurden verschiedene unterstützende Technologien entwickelt. Diese Technologien sollen beispielsweise bei der Führung der chirurgischen Instrumente und der Erkennung anatomischer Strukturen helfen, zudem sollen sie eine insgesamt verbesserte Wahrnehmung der Operationsbedingungen ermöglichen.

Alle bekannten Lösungen erfordern eine physische Interaktion des Instruments mit dem Gewebe und unterscheiden sich technisch im Wesentlichen durch den Ort und die Art der Sensoren, die für die Messung beispielsweise von Kraft, Drehmoment, Druck, Impedanz und Vibrationen verwendet werden.

Bei der direkten Abtastung werden in der Regel eine mit Sensoren versehene Instrumentenspitze, eine spezielle Sonde oder eine Anordnung von Messwandlern in direktem Kontakt mit der Zielstruktur verwendet. Nachteilig sind die konstruktions- und fertigungstechnischen Herausforderungen, die beispielsweise die Integration von Sensoren an dem Instrument selbst mit sich bringen, ohne die ursprüngliche Funktionalität oder Qualität des Instruments zu beeinträchtigen, sowie die Einhaltung von Sterilisations- und Biokompatibilitätsanforderungen.

Indirekte Messungen hingegen sind dadurch gekennzeichnet, dass sich ein einzelner Messwertwandler außerhalb des Körpers aber in der Nähe des Betätigungsmechanismus des Instruments befindet. Dadurch werden einige Einschränkungen der direkten Abtastung gemildert, aber die räumliche Auflösung ist auf eine punktuelle Bewertung beschränkt und die Messgenauigkeit und - konsistenz ist begrenzt.

Bekannte visuelle Technologien sollen die optische Wahrnehmung des Chirurgen unterstützen, auch wurden sensorbasierte Lösungen entwickelt, um beispielsweise den Tastsinn des Chirurgen zu unterstützen und die Darstellung von Informationen in Form von akustischem Feedback wurde eingeführt, um den Hörsinn des Chirurgen zu unterstützen.

So wird derzeit in der MIS, beispielsweise bei laparoskopischen oder robotergestützten Eingriffen, eine visuelle Unterstützung durch RGB-Kameras bereitgestellt, indem eine endoskopische Kamera durch einen winzigen Einschnitt in den Operationsbereich eingeführt wird.

Diese unterstützenden Informationen werden in Form von Echtzeitvideos mit hoher Qualität und Auflösung auf einem Monitor dargestellt und können auch mit Tiefeninformationen kombiniert werden, um eine dreidimensionale Darstellung des Operationsgebiets zu erhalten.

Inzwischen ist die Nachbearbeitung und Analyse der so entstandenen Videos Teil eines neuen medizintechnischen Forschungsgebiets namens *Surgical Data Science* (SDS), das verschiedene Bereiche einbeziehen kann, wie beispielsweise chirurgische Entscheidungshilfe, chirurgische Ausbildung und kontextbezogene Unterstützung wie die Überwachung von Eingriffen, die Vorhersage der verbleibenden Operationsdauer, die Überwachung von Eingriffen zur Erleichterung der Zeitplanung, die Überwachung von Eingriffen zur Vorhersage des Ressourcenbedarfs, die Erkennung chirurgischer Phasen, die Einbeziehung kollaborativer Roboter, die Vorhersage schlechter Ergebnisse, die Warnung vor möglichen Problemen und die retrospektive Identifizierung der Ursachen schlechter Ergebnisse.

Der größte Nachteil der Videospeicherung und -analyse ist die große Menge an Speicherplatz für Videoaufnahmen und der begrenzte dynamische Bereich, den Videos bieten können. Aufgrund der niedrigen Bildrate von Videos beschränken sich die Informationen, die aus dieser Art von Daten gewonnen werden können, in der Regel auf die vom Menschen wahrnehmbaren Informationen. Es ist jedoch bekannt, dass bei biologischen und mechanischen Prozessen die gewonnenen Daten auch Einblicke in Bereiche geben können, die mit den menschlichen Sinnen nur schwer oder gar nicht wahrgenommen werden können.

Ein weiterer Nachteil insbesondere endoskopischer Videos ist, dass der chirurgische Eingriff in der Regel nur aus einer Perspektive erfasst wird. Bei der laparoskopischen Chirurgie wird nämlich nur eine einzige endoskopische Kamera im Körper platziert, was die Überwachung des chirurgischen Vorgangs auf diese eine Perspektive beschränkt.

Zusätzlich zu RGB-Kameras werden Chirurgen bei manchen Arten von minimalinvasiven Eingriffen durch weitere bildgebende Verfahren wie beispielsweise Magnetresonanztomographie, Röntgen, Computertomographie und Ultraschall unterstützt.

Je nach angewandtem Verfahren kann die visuelle Wahrnehmung des Chirurgen auch durch die Kombination dieser Bildgebungstechnologien mit Instrumentenverfolgungs- und Navigationssystemen wie Optical Tracking und Electromagnetic Tracking verbessert werden, um die visuelle Rückmeldung über die Position des Instruments sowie das anvisierte Organ oder die anatomische Struktur hervorzuheben.

Tatsächlich beschränkt sich die Mehrzahl der aktuellen Entwicklungen, die versuchen die verminderte sensorische Wahrnehmung des Chirurgen in der MIS zu kompensieren, auf die Verbesserung der visuellen Wahrnehmung.

Doch die Anzeige von Informationen auf einem Bildschirm ist häufig keine ideale Lösung, da die Chirurgen ihren Blick vom OP-Bereich abwenden müssen, um auf den entsprechenden Monitor zu schauen und die dort zur Verfügung gestellten Informationen zu erhalten. Auch die als zusätzliche Hilfe gedachte dreidimensionale Darstellung von Informationen ist nicht immer intuitiv und benötigt in der Regel ein vorheriges Training und Erfahrung, um korrekt interpretiert zu werden.

Zudem verfügen die während der Operation zur Verfügung gestellten Echtzeitvideos nicht über eine Tonspur, geben also keine zusätzlichen akustischen Informationen, die den Operationsverlauf weiter klären könnten. Trotzdem benötigen sie für eine mögliche Nachauswertung viel Speicherplatz und sind rechenintensiv in der Verarbeitung.

Nicht zuletzt haben Videoaufnahmen den großen Nachteil, dass sie immer nur ein sehr eingeschränktes Blickfeld (Field of View; FoV) haben und - im Gegensatz zu anderen bildgebenden Verfahren - nicht unter die Oberfläche von Strukturen blicken können. Die Sichtlinie (Line of Sight; LoS) des Videos und somit die des Chirurgen endet an der ersten blickdichten Oberfläche. In beiden Fällen (FoV, LoS) können mit den bekannten Videoverfahren Interaktionen außerhalb der Bildbereiche vom Operateur nicht erkannt und vom System nicht erfasst werden und entsprechend nicht ausgewertet werden.

Entsprechend ergibt sich ein großer Bedarf für weitere Sensortechniken, um zusätzliche Daten und Informationen bereitstellen zu können, die insbesondere auch von Videosystemen nicht bereitgestellt werden können.

Im Stand der Technik sind ergänzend oder alternativ zur Videoüberwachung verschiedene sensorgestützte Entwicklungen bekannt, um speziell den fehlenden Tastsinn des Chirurgen bei minimalinvasiven Eingriffen zu ersetzen. Diese Lösungen nutzen verschiedene Arten von Sensortechniken zur Messung von Kraft, Drehmoment, Druck, Impedanz, Vibrationen oder zur Erfassung jeglicher Art von taktilen Informationen. Auch verschiedene Arten von Sonden zur Messung von Gewebemerkmalen wurden vorgeschlagen.

Eine breitere Akzeptanz eines dieser Systeme ist im klinischen Bereich bisher weder erkennbar noch zukünftig absehbar. Denn häufig sind entsprechende Systeme äußerst kostenintensiv und können auch nicht mit bekannten und verfügbaren Standard-Instrumenten kombiniert werden.

Bei den meisten Systemen sind nämlich die notwendigen Sensoren am distalen Ende des Instruments eingebettet, was die Entwicklung proprietärer Instrumente erfordert, die auf das jeweilige System abgestimmt sein müssen. Bei solchen Instrumenten müssen zudem die Sensoren mit in den Körper des Patienten einführt werden. Auch werden in der Regel zusätzliche Verkabelungen und aktive Komponenten notwendig, die ebenfalls in das Instrument eingebettet werden müssen.

Insbesondere die auditive Unterstützung bei minimalinvasiven Eingriffen ist noch immer unzureichend erforscht. Dennoch wurden einige Versuche unternommen, Technologien zur Unterstützung der Informationsdarstellung und zur Verbesserung des Hörsinns bei MIS zu entwickeln, die sich auf zwei Bereiche konzentrieren: die auditive Darstellung und die Sonifikation.

Die auditive Darstellung wird hauptsächlich in Kombination mit bildgesteuerten Interventionssystemen bei einer Vielzahl von minimalinvasiven Eingriffen eingesetzt, wie beispielsweise bei der Platzierung von Nadeln (unter anderem im Rahmen einer Radiofrequenz-Nadelablation oder einer Nadelbiopsie), beim Bohren des Schläfenbeins, bei der volumetrischen Geweberesektion oder beim telerobotischen Spannen von Nähten.

Dazu werden Daten aus verschiedenen Quellen - darunter beispielsweise die von dem bei der Intervention genutzten Navigationssystem gelieferten Entfernungsinformationen - als sich ändernde Parameter eines Schallgenerators abgebildet, der ein entsprechendes akustisches Feedback erzeugt. Zu den auditiven Darstellungsmethoden gehören beispielsweise:
1) Warnmeldungen, bei denen Töne abgespielt werden, wenn die ermittelten Werte einen vorbestimmten Schwellenwert erreichen oder überschreiten oder unterschreiten;
2) auditive Symbole, bei denen es sich um bekannte Klänge handelt, um Informationen über Ereignisse in Analogie zu alltäglichen Geräuschen zu vermitteln; und
3) Parameterzuordnungen, die kontinuierliche Änderungen in einem Datensatz in kontinuierliche Änderungen in Audioparametern, beispielsweise der Tonhöhe oder Lautstärke, übersetzen.

Die Sonifikation konzentriert sich auf die Verwendung von nicht-sprachlichen Audiodaten, um die wichtigsten physikalischen Parameter eines Tons wie Frequenz (Tonhöhe), Dauer und Intensität mit einer wichtigen Eigenschaft des darzustellenden Signals akustisch zu verbinden, wie beispielsweise Klangfarbe, Rhythmus und Harmonie. In den Bereichen Herzfrequenzanalyse, Biorhythmusanalyse, Elektrokardiographie, molekulare Sequenzen, zelluläre Kinetik, medizinische Bilddiagnose und Elektroenzephalographie finden mehrere Forschungsstudien zur Sonifikation statt.

Obwohl diese bekannten Methoden dem Chirurgen eine bessere Wahrnehmung während eines minimalinvasiven Eingriffs ermöglichen können, sind diese Methoden für minimalinvasive Eingriffe weniger erforscht und auch weniger entwickelt. Bislang können sie den fehlenden Teil des Hörsinns bei MIS nicht oder zumindest nicht vollständig kompensieren.

Es ist daher Aufgabe der Erfindung, eine Vorrichtung und ein Verfahren zur Verfügung zu stellen, die die erwähnten Nachteile nicht aufweisen.

Es ist dabei insbesondere eine Aufgabe der Erfindung eine Vorrichtung und ein Verfahren zur Verfügung zu stellen, die insbesondere bei oder im Zusammenhang mit minimalinvasiven Eingriffen geeignet sind, die fehlenden sensorischen Eindrücke durch eine geeignete Rückmeldung basierend auf dem Erfassen und Auswerten von vibroakustischen Signalen zu kompensieren.

Es ist weiterhin eine Aufgabe der Erfindung, basierend auf der erfinderischen Vorrichtung und dem erfinderischen Verfahren, ein chirurgisches Überwachungssystem zur Verfügung zu stellen, das kosteneffizient und einfach in der Anwendung ist. Das System soll zudem einen großen dynamischen Bereich bieten, der über die menschliche Wahrnehmung hinausgeht, und somit vielfältige Beobachtungsmöglichkeiten eines Prozesses liefern kann.

Gelöst wird diese Aufgabe durch eine Vorrichtung mit den Merkmalen des Anspruchs 1 und ein computerimplementiertes Verfahren mit den Merkmalen des Anspruchs 4. Vorteilhafte Ausgestaltungen sind jeweils Gegenstand der abhängigen Ansprüche. Es ist darauf hinzuweisen, dass die in den Ansprüchen einzeln aufgeführten Merkmale auch in beliebiger und technologisch sinnvoller Weise miteinander kombiniert werden können und somit weitere Ausgestaltungen der Erfindung aufzeigen.

Die vorliegende Erfindung bezieht sich entsprechend auf eine Vorrichtung und ein computerimplementiertes Verfahren zur Erfassung und Auswertung vibroakustischer Signale. Die Vorrichtung ist geeignet zur Verwendung mit medizinischen Instrumenten.

Das erfindungsgemäße Verfahren betrifft beispielsweise die Aufarbeitung der vibroakustischen Signale zur Audifikation und Augmentation sowie zur Bewertung, zum Monitoring und zur Dokumentation medizinischer Verfahren oder medizinischer Aufgaben. Medizinische Verfahren können beispielsweise laparoskopische und robotergestützte Chirurgie, Arthroskopie oder Nadeleingriffe umfassen, medizinische Aufgaben können beispielsweise Palpation oder Ablation umfassen, um nur einige Beispiele in dieser nicht abschließenden Aufzählung zu nennen.

Unter "Audifikation" (im Englischen *audification*) versteht man im weiteren Sinne die Übersetzung eines Datensatzes in ein akustisch wahrnehmbares Ereignis. Der Ursprung des Datensatzes kann unterschiedlich sein und muss zunächst selbst kein mit unseren menschlichen Sinnen wahrnehmbares Ereignis betreffen. Der Ursprung des Datensatzes kann somit beispielsweise im nicht hörbaren Frequenzbereich oder unterhalb der menschlichen Wahrnehmungsschwelle für Vibrationen liegen. Der ursprüngliche Datensatz kann zunächst auch völlig losgelöst von einem hörbaren Ereignis sein, es lassen sich grundsätzlich beliebige Datensätze beliebiger Ereignisse mit der Audifikation hörbar und somit erlebbar machen.

Die Audifikation erfolgt durch den Anschluss einer oder mehrerer erfindungsgemäßer Vorrichtungen an einem oder mehreren medizinischen Instrumenten. Unter den Begriffen "medizinisches Instrument" oder "Instrument" sollen hierbei alle medizinischen, chirurgischen oder interventionellen Werkzeuge und Instrumente zusammengefasst werden, die während eines medizinischen Verfahrens, also beispielsweise einer nicht invasiven Intervention oder einer konventionellen offenen Operation oder einer minimal invasiven Intervention, jeweils auch im Rahmen einer Diagnostik, von den durchführenden Personen benutzt werden.

Die erfindungsgemäße Vorrichtung umfasst eine Signalerfassungsvorrichtung, eine Signal-/Datenverarbeitungsvorrichtung und optional eine Ausgabevorrichtung.

Die erfindungsgemäße Vorrichtung ist vorzugsweise im Sinne einer Add-on Vorrichtung mit einem medizinischen Instrument verbindbar. Hierzu ist an der Signalerfassungsvorrichtung ein Kopplungselement vorgesehen, das vorzugsweise an bekannte Standardverbindungen, wie beispielsweise Luer-Lock-Verbindungen angepasst ist. Das Kopplungselement kann aber auch universell für verschiedene Anschlüssen vorgesehen sein.

Die erfindungsgemäße Vorrichtung kann alternativ auch in dem jeweiligen medizinischen Instrument implementiert sein. Dies ist insbesondere dann vorteilhaft, wenn die Vorrichtung beispielsweise in der Roboter-unterstützten-Chirurgie (RAS) eingesetzt wird und Teil des Roboter-Moduls ist.

Das Kopplungselement kann eine formschlüssige Öffnung zur Einbringung eines medizinischen Instruments oder eine gegebenenfalls kraftschlüssig wirkende Vorrichtung, beispielsweise in Form eines Klammerelements, mit dem die erfindungsgemäße Vorrichtung an einem medizinischen Instrument anbringbar ist, umfassen.

Optional umfasst die erfindungsgemäße Vorrichtung ein Adapterelement, das vorgesehen ist, eine passgenaue, also eine form-, reib- und/oder kraftschlüssige Verbindung zwischen dem medizinischen Instrument und dem Kopplungselement herzustellen, sofern hier eine solche weitere Anpassung notwendig ist.

Kopplungselement und Adapterelement sind vorgesehen, vibroakustische Signale von dem medizinischen Instrument möglichst störungsfrei an einen Sensor zu übertragen.

Wenn nämlich ein Teil des medizinischen Instruments, also beispielsweise der Schaft oder zunächst die Spitze, mit einem wie nachfolgend definierten Gewebe in Kontakt kommt, wird an der Kontaktstelle eine mechanische Schwingung erzeugt. Die Schwingung breitet sich auf passive Weise, ohne dass eine aktive oder aktivierende Komponente erforderlich ist, als vibroakustische Welle durch das gesamte Instrument und somit zu jedem beliebigen Punkt der physischen Oberfläche des medizinischen Instruments aus. Dies umfasst auch eine Ausbreitung auf ein mit dem medizinischen Instrument verbundenes Objekt wie beispielsweise weitere Instrumente oder eine Ausbreitung auf eine sonstige Struktur.

Durch das Anbringen eines Messsystems in Form der erfindungsgemäßen Vorrichtung ist es entsprechend möglich, die sich an dem Instrument ausbreitende Schwingung, also die vibroakustischen Signale, als eine physikalische Größe zu messen und eine quantitative und qualitative Bewertung vorzunehmen.

Die gemessene Größe steht dabei direkt in Verbindung mit der Wechselwirkung des Instruments mit dem Gewebe oder dem Gebrauch des Instruments an sich, beispielsweise bei der Interaktion der beiden Schneiden einer laparoskopischen Schere oder auch dem reinen Aufnehmen oder Ablegen eines Instruments vor oder nach dem Gebrauch.

Sofern während eines Eingriffs mehrere Instrumente verwendet werden, was bei den meisten medizinischen Eingriffen die Regel ist, so kann an jedes Instrument eine erfindungsgemäße Vorrichtung angeschlossen werden, um vibroakustische Signale zu messen, die aus der Interaktion zwischen dem Instrument und der Umgebung und insbesondere zwischen dem Instrument und dem Gewebe entstehen.

Unter einem "Eingriff" sollen dabei sowohl Operationen im Sinne der klassischen offenen Chirurgie als auch minimal invasive Eingriffe verstanden werden. Denn auch wenn sich die vorliegende Erfindung insbesondere an den Anforderungen minimal invasiver Eingriffe orientiert, so kann die Anwendung der Erfindung bei Eingriffen in jeglichem medizinischen Umfeld vorteilhaft sein.

Unter vibroakustischen Signalen, nachfolgend auch "VA-Signale", sollen dabei solche Signale verstanden werden, die auf einer Schwingung des medizinischen Instruments beruhen, wobei die Schwingung und somit das vibroakustische Signal das Ergebnis der Interaktion des Instrumentes mit seiner Umgebung, also insbesondere mit dem jeweils umliegenden Gewebe, ist.

Insbesondere ist bei der bevorzugten Ausführungsform der Erfindung keine aktive Anregung des medizinischen Instruments vorgesehen, was jedoch in alternativen Ausführungsformen denkbar wäre.

Der Begriff "Gewebe" ist breit zu verstehen und umfasst alle natürlichen, biologischen oder künstlichen physischen Strukturen, Oberflächen, Gewebe oder Objekte, die mit dem Instrument interagieren können.

Die erfindungsgemäße Vorrichtung umfasst entsprechend als Teil der Signalerfassungsvorrichtung weiterhin mindestens einen Sensor zur Erfassung vibroakustischer Signale, insbesondere einen Akustik- oder Vibrationssensor, also einen schallwandelnden Sensor, wie beispielsweise ein Mikrofon.

Das Kopplungselement ist entsprechend sowohl mit dem Instrument als auch direkt oder indirekt mit dem mindestens einen Sensor schallübertragend verbunden und geeignet, vibroakustische Signale möglichst störungs- und verlustfrei von dem Instrument an den mindestens einen Sensor zu übertragen.

Bei einer direkten Verbindung ist der mindestens eine Sensor direkt, also insbesondere kraft-, form- und/oder reibschlüssig, mit dem Kopplungselement verbunden, wobei hier vorzugsweise ein Piezo- oder Kristallmikrofon als Sensor eingesetzt werden. Der Einsatz sonstiger schallwandelnder Sensoren ist denkbar.

Bei einer indirekten Verbindung ist das Kopplungselement nicht direkt mit einem Sensor, sondern vorzugsweise nicht dauerhaft aber zumindest kraft- oder reibschlüssig, also wirkungsübertragend, mit einer Membran verbunden, die die vibroakustischen Signale in einen sich an die Membran anschließenden Schallraum überträgt.

Eine solche Anordnung mit einer Membran und einem Schallraum wird nachfolgend auch als mechanischer Verstärkungsmechanismus bezeichnet.

Die vibroakustischen Signale weisen entsprechend eine Impuls-Komponente auf, die, in der Regel über das Kopplungselement, in axialer Richtung, also senkrecht zur Membran, wirkt und die zu einer Anregung beziehungsweise Auslenkung der Membran führt.

Die vibroakustischen Signale weisen ein zusätzliches Biegemoment im Kopplungspunkt des Kopplungselements mit der Membran auf, wobei sowohl durch die axiale Impuls-Komponente als auch durch das Biegemoment eine Auslenkung der Membran und damit eine Schallwelle beziehungsweise eine Druckwelle in dem sich an die Membran anschließenden Schallraum erzeugt wird.

Dabei kann das Instrument selbst als Hebelarm für das resultierende Moment wirken und die Auslenkung der Membran entsprechend verstärkt.

Das Kopplungselement ist in diesem Fall also geeignet, vibroakustische Signale möglichst störungs- und verlustfrei von dem Instrument an die Membran zu übertragen.

Die Anregung beziehungsweise Auslenkung der Membran erzeugt also eine Schallwelle beziehungsweise Druckwelle in dem sich an die Membran anschließenden Schallraum.

Der Schallraum ist als Hohlraum vorgesehen und vorzugsweise so ausgestaltet, dass er sich von der Membran hin zum Sensor verjüngt. Der Sensor nimmt entsprechend die vibroakustischen Signale als durch die Membran verstärkte Schallwellen aus dem Schallraum auf. Die Membran ist entsprechend geeignet, die vibroakustischen Signale zu verstärken, wobei der Schallraum zu einer zusätzlichen Verstärkung des Signals führt.

Grundsätzlich ist es auch denkbar, dass die vibroakustischen Signale direkt, also ohne eine zwischengeschaltete Membran, vom Kopplungselement in den Schallraum übertragen werden.

Der Schallraum kann optional mit einem gasförmigen oder flüssigen Fluid gefüllt sein, um gewünschte Übertragungseigenschaften zu schaffen.

An dem Schallraum ist der mindestens eine Sensor vorgesehen. Vorzugsweise wird der Schallraum durch den Sensor begrenzt, es sind jedoch auch Ausführungsformen denkbar, bei denen der Sensor innerhalb des Schallraums vorgesehen ist. Auch sind Ausführungsformen denkbar, bei denen der Sensor außerhalb des Schallraums jedoch vorzugsweise mit diesem verbunden vorgesehen ist.

Für die beschriebene indirekte Verbindung ist vorzugsweise ein luftgebundenes Mikrofon mit einer zweiten Membran als wandelndem Element vorgesehen.

Die zweite Membran des Sensors, also beispielsweise die des luftgebundenen Mikrofons, besitzt vorzugsweise einen signifikant kleineren Durchmesser als die Membran des Schallraums. In Wirkeinheit mit dem sich vorzugsweise zum Sensor hin verjüngenden Schallraum führen die unterschiedlichen Durchmesser der Membranen zu einer deutlichen Verstärkung der Auslenkung der zweiten Membran.

Die Anordnung führt durch die Verstärkung aufgrund der Membran-Kombination sowie gegebenenfalls aufgrund der Hebelwirkung des Instruments zu einer erheblich gesteigerten Empfindlichkeit der Vorrichtung bereits gegenüber minimalen strukturellen Vibrationen des Instruments, die aus Interaktions-Kräften am Instrument resultieren.

Neben diesen Sensoren, die geeignet sind, vibroakustische Signale zu erfassen, können weitere Sensoren an der Signalerfassungsvorrichtung vorgesehen sein, insbesondere Beschleunigungssensoren, Gyroskope, Kraftsensoren, Reibungssensoren, Vibrationssensoren oder auch Drucksensoren. Auch ist die Implementierung von optischen Sensoren denkbar, insbesondere auch von Interferometern zur Vibrationsmessung. Durch die verschiedenen Sensoren können verschiedene Arten von optischen und mechanischen Messgrößen erfasst und verarbeitet werden.

Die von dem mindestens einen Sensor erfassten vibroakustischen Signale werden optional auf der analogen Stufe weiterbearbeitet, sie werden also optional einer analogen Signalvorverarbeitung zugeführt. Die Weiter- beziehungsweise Vorverarbeitung auf der analogen Stufe kann insbesondere eine Filterung und Verstärkung des analogen Signals umfassen.

Anschließend können die Daten digitalisiert und an die Daten-/Signalverarbeitungsvorrichtung übertragen werden.

Hierzu umfasst die erfindungsgemäße Vorrichtung einen analog-digital-Wandler, der die analogen Signale in digitale Daten umwandelt. Ein solcher Wandler kann auch Teil des zumindest einen Sensors sein.

Zur Übertragung beziehungsweise zum Austausch der digitalisierten Daten umfasst die Signalerfassungsvorrichtung eine Kommunikationseinheit.

Die von dem Sensor erfassten und gegebenenfalls analog bearbeiteten Signale werden digitalisiert als digitales Signal beziehungsweise als digitale Daten an die Daten-/Signalverarbeitungsvorrichtung übertragen, die das Signal beziehungsweise die Daten weiterverarbeitet. Die Daten-/Signalverarbeitungsvorrichtung kann in der erfindungsgemäßen Vorrichtung einhäusig zusammen mit der Signalerfassungsvorrichtung eingebettet oder kabelgebunden oder kabellos mit dieser verbunden sein.

Die Daten-/Signalverarbeitungsvorrichtung ist entsprechend vorgesehen, das computerimplementierte Verfahren zur Verarbeitung der digitalisierten vibroakustischen Signale auszuführen und das Ergebnis der Verarbeitung zur Verfügung zu stellen und beispielsweise an die Ausgabevorrichtung weiterzugeben, um eine entsprechend gewünschte Ausgabe auszulösen.

Anders ausgedrückt umfasst die Daten-/Signalverarbeitungsvorrichtung ein nichttransitorisches computerlesbares Medium, das Befehle enthält, die, wenn sie von mindestens einem Prozessor ausgeführt werden, den mindestens einen Prozessor veranlassen, Operationen auszuführen, die es ermöglichen, vibroakustische Signale zu verarbeiten und das Ergebnis zur Übertragung an die Ausgabevorrichtung bereitzustellen oder direkt weiterzuleiten, um die Ausgabevorrichtung zur Speicherung oder Ausgabe einer entsprechenden Rückmeldung an den Anwender zu veranlassen.

Die Ausgabevorrichtung ist entsprechend vorgesehen, das Ergebnis der Daten-/Signalverarbeitungsvorrichtung auszugeben oder aber zur späteren Verwendung zu speichern. Die Ausgabe kann beispielsweise in Form eines optischen, eines akustischen oder eines haptischen Signals oder durch eine Kombination aus einem oder mehreren dieser Signale erfolgen.

Die Signalerfassungsvorrichtung und die Daten-/Signalverarbeitungsvorrichtung stellen neben dem computerimplementierten Verfahren die wesentlichen technischen Elemente der erfindungsgemäßen Vorrichtung dar. Diese Elemente können unterschiedlich angeordnet und gruppiert sein.

So können beispielsweise die Signalerfassungsvorrichtung und die Daten-/Signalverarbeitungsvorrichtung in einem Gehäuse vorgesehen sein.

Bei bestimmten Ausführungsformen kann auch die optionale Ausgabevorrichtung Teil der einhäusigen Ausführung sein.

Es sind jedoch auch Ausführungsformen der erfindungsgemäßen Vorrichtung denkbar, bei denen die Vorrichtung aus mehreren einzelnen Elementen besteht. Dabei meint "einzeln", dass diese einzelnen Elemente gerade nicht in einem einzigen Gehäuse angeordnet sind, sondern zumindest eines der Elemente mit den übrigen Elementen nur über ein Kabel oder kabellos verbunden ist.

So sind beispielsweise Ausführungsformen denkbar, bei denen eine kabelgebundene oder kabellose Übertragung der digitalisierten Daten von der Signalerfassungsvorrichtung an eine räumlich separierte Daten-/Signalverarbeitungsvorrichtung erfolgt.

Ebenso sind Ausführungsformen denkbar, bei denen beispielsweise nur die Signalerfassungsvorrichtung und eine Ausgabevorrichtung in einem Gehäuse angeordnet sind, sodass der Benutzer die Rückmeldung über die Ausgabevorrichtung direkt am Erfassungsort erhält, die Datenverarbeitung jedoch räumlich getrennt in der Daten-/Signalverarbeitungsvorrichtung erfolgen kann.

Die Ausgabevorrichtung kann Teil der erfindungsgemäßen Vorrichtung sein, das Ergebnis der Daten-/Signalverarbeitungsvorrichtung kann jedoch auch über eine externe Ausgabevorrichtung ausgegeben werden, die nicht Teil der erfindungsgemäßen Vorrichtung ist. Mögliche Ausgabevorrichtungen können Computer, Smartphones, Laptops, Tablets oder sonstige Geräte sein, die die entsprechende Eingabe der Daten-/Signalverarbeitungsvorrichtung in eine gewünschte Ausgabe umwandeln können.

Im Falle der RAS ist es denkbar, dass das Ergebnis der Datenverarbeitung direkt zur Steuerung des RAS-Systems und somit zur weiteren Durchführung der Operation genutzt wird, also in diesem Sinne keine Ausgabe in Form eines Signals erfolgt, sondern eine Weiterverarbeitung des Ergebnisses der Daten-/Signalverarbeitungsvorrichtung erfolgt.

Entsprechend sind im Sinne dieser Erfindung sowohl die Ausgabe von Daten als auch die Beschaffenheit der Ausgabevorrichtung weit zu verstehen, indem die Ausgabe nicht zwingend beispielsweise in akustischer, haptischer oder visueller Form erfolgen muss, sondern die Ausgabe auch beispielsweise zur weiteren Steuerung oder Planung einer Operation oder der Bewegung eines Roboters genutzt werden kann.

Die Ausgabevorrichtung kann entsprechend auch eine Speicherung oder Datenweiterleitung umfassen.

Die erfindungsgemäße Vorrichtung kann direkt oder indirekt an das zu messende beziehungsweise zu audifizierende Instrument angeschlossen werden.

Ein direkter Anschluss führt zu einer direkten Messung. Unter einer direkten Messung ist zu verstehen, dass die Vorrichtung direkt an das zu audifizierende Instrument angeschlossen wird. Ein Beispiel hierfür ist die Audifikation einer Biopsie-Nadel, eines Palpierstabes, eines Palpierhakens oder einer Tastsonde.

Ein indirekter Anschluss führt zu einer indirekten Messung. Unter einer indirekten Messung ist zu verstehen, dass ein erstes zu audifizierendes Instrument gemessen wird, indem ein zweites Instrument gemessen wird, mit dem das erste Instrument schall- beziehungsweise vibrationsübertragend verbunden ist.

Ein Beispiel hierfür ist die Audifikation eines laparoskopischen Greifers, indem die erfindungsgemäße Vorrichtung nicht direkt an den laparoskopischen Greifer, sondern an den Trokar angeschlossen wird, in den der Greifer eingeführt wird.

Die interessierenden vibroakustischen Signale werden also nicht direkt an dem laparoskopischen Greifer, sondern indirekt an dem Trokar gemessen, auf den diese vibroakustischen Signale von dem laparoskopischen Greifer übertragen werden.

Beispiele für Interventionen, die je nach Art der MIS audifiziert werden können, sind unter anderem laparoskopische und roboterassistierte Chirurgie (RAS), wie Palpation, Greifen, Schneiden, Nähen, Knoten-/Nahtfestziehen, Ausreißen/Ausfransen von Nahtmaterial, Ablation, HF-Koagulation, Pulsatilität, Kollision zwischen Werkzeugen, Werkzeugwechsel/Platzierung eines Werkzeugs durch den Trokar-Port oder die Kollision eines Werkzeugs mit einem bestimmten Gewebe außerhalb des Sichtfelds des Endoskops.

Eine andere Kategorie von Interventionen betrifft Nadeleingriffe wie beispielsweise Nadelpunktion, Nadel-Gewebe-Gewebe-Passage (darunter Punktion einer Organgrenze, Punktion einer Tumorgrenze, Erreichen eines leeren oder flüssigen Hohlraums, Perforation oder Punktion eines Gefäßes, Biopsie-Probenentnahme).

Eine weitere Kategorie von Interventionen betrifft Eingriffe mit Führungsdrähten/Kathetern wie beispielsweise Perforation der Gefäß-/Arterienwand, Wandreibung, Blockierung, Pulsation/Fluss, Werkzeugbedienung (unter anderem Fräsen, Schneiden, etc.), Stent-Entfaltung, Kontakt Stent-Gefäßwand, Ballon-Inflation oder auch Kontakt Ballon-Gefäßwand.

Eine weitere Kategorie betrifft die Arthroskopie mit Palpation, Klopfen, Schaben, Fräsen, Schrauben, Bohren, Schneiden oder Glätten.

### ALLGEMEINE VERARBEITUNGSMETHODIK

Die Erfindung betrifft auch ein computerimplementiertes Verfahren zur Verarbeitung der erfassten vibroakustischen Signale.

Das VA-Signal, das aus der Interaktion zwischen einem medizinischen Instrument und dem Gewebe resultiert, weist Eigenschaften auf, die eine Verarbeitung mit standardisierten Signalverarbeitungsalgorithmen, die normalerweise bei der Sprach-/Audioverarbeitung eingesetzt werden, sehr schwierig machen. Merkmale des Signals, die die Analyse komplex machen, sind unter anderem:
Die Nicht-Stationarität: Die statistischen und spektralen Merkmale eines VA-Signals, das aus den dargestellten Interaktionen zwischen medizinischem Instrument und Gewebe resultiert, können sich innerhalb von Millisekunden abrupt ändern. Dies führt zu einer Reihe von unterschiedlichen Dynamiken, die in einem einzigen Interaktionsereignis innerhalb kürzester Zeit von transient zu oszillierend und dann wieder zu transient übergehen können. Die Verarbeitung von Transienten mit bekannten Signalverarbeitungsalgorithmen für Sprache und Ton ist sehr schwierig, ebenso wie die Verarbeitung von sehr unstetigen und hochgradig nicht-stationären Signalen.

Viele Dinge, die in einem Interaktionsereignis passieren, führen zu sogenannten Mikrogeräuschen, also Geräuschen, die weniger als 200 ms andauern können und die das menschliche Gehör nicht wahrnehmen kann. Die Punktion einer Gewebe-Gewebe-Passage während des Einstichs der Veres Nadel dauert beispielsweise nur etwa 50 ms.

Das VA-Signal kann während eines medizinischen Eingriffes auf verschiedenste Weise moduliert werden. Bei diesen Modulationen handelt es sich in der Regel um nichtlineare Dynamiken, die mit bekannten Standardalgorithmen zur Audio- oder Sprachsignalverarbeitung nur schwer zu verarbeiten sind.

Eine zusätzliche Komplexität liegt in den unterschiedlichen Energieniveaus, die in einem einzigen Interaktionsereignis enthalten sein können. Wenn beispielsweise ein Gewebe ertastet wird, kann die Energie, die im Moment des ersten Kontakts mit dem Gewebe erzeugt wird, mehr als das Zehnfache der Energie der effektiven beziehungsweise eigentlichen Ertastung betragen. Beim Beispiel der Veres-Nadel kann die interne, mechanische Interaktion der Nadel durch den ausgelösten Federmechanismus eine 50-mal höhere Energie haben als die beim Einsatz entstehende Reibung oder der Einstich selbst. Die Verarbeitung von Signalen mit einer solchen Varianz von Energien ist ebenfalls komplex und erfordert die Implementation neuer, erfinderischer Prozesse.

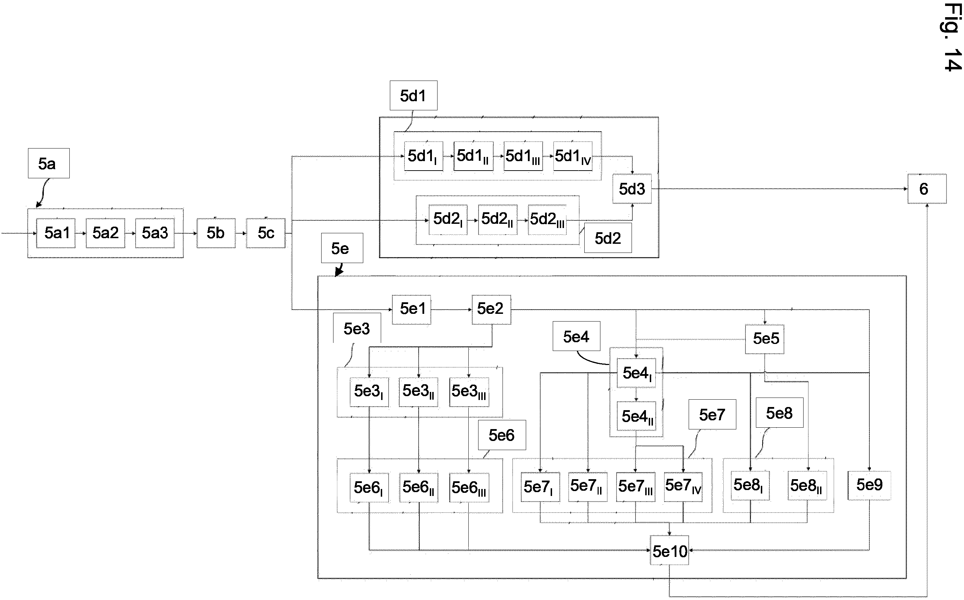

Das erfindungsgemäße computerimplementierte Verfahren wird nachfolgend in der Figur 14 dargestellt und hier etwas allgemeiner zusammengefasst. Der genaue Verfahrensablauf lässt sich der Figur 14 entnehmen.

Das erfindungsgemäße computerimplementierte Verfahren umfasst in einem ersten Schritt grundsätzlich das Bereitstellen vorhandener digitalisierter vibroakustischer Signale.

Der grundsätzliche Verfahrensablauf des erfindungsgemäßen computerimplementierten Verfahrens umfasst die folgenden Schritte:
(0) Bereitstellen eines vorhandenen digitalisierten vibroakustischen Signals;
(I) Signalvorverarbeitung des vibroakustischen Signals; und
(II) Identifizierung von Segmenten/Regionen von Interesse; und
(III) Audifikation; und/oder
(IV) Klassifizierung der Segmente/Regionen von Interesse.

Optional erfolgt in einem weiteren Schritt (V) beispielsweise die Ausgabe, Weiterverarbeitung, Speicherung etc. der klassifizierten Segmente/Regionen.

Das Verfahren umfasst eine Vorverarbeitung der digitalisierten VA-Signale. Dieser Schritt soll die verschiedenen Störgeräusche im VA-Signal abschwächen und die Interaktionsereignisse der Instrumentenspitze beziehungsweise des Schafts verstärken. '

Zunächst werden die kontinuierlichen Komponenten (DC) aus dem VA-Signal entfernt. Dann wird ein Schritt zur Reduzierung des Hintergrundrauschens durchgeführt. Dies kann zum Beispiel durch spektrale Gating-Methoden erfolgen. Schließlich wird ein Schritt zur Signalverbesserung durchgeführt, um die Dynamik und die Frequenzkomponenten im Zusammenhang mit den Interaktionen zu verbessern. Dies kann mit linearen Filtern, adaptiven Filtern oder mit Wavelet-basierten Filtern geschehen.

Bei Wavelet-basierten Filtern kann die diskrete Wavelet-Transformation (DWT) verwendet werden, um das Signal in verschiedene Skalen zu zerlegen und dann das Signal anhand der Skalen zu rekonstruieren, in denen Wechselwirkungen mit hoher Energie auftreten.

In einem nächsten Schritt nach der Vorverarbeitung kann die Identifizierung von Segmenten/Regionen von Interesse erfolgen. Dieser Schritt kann verwendet werden, um interessierende Regionen im Signal zu segmentieren, in denen wichtige Interaktionsereignisse zwischen medizinischem Instrument und Gewebe/Oberfläche auftreten. Dies kann dazu dienen, eine Prüfung oder Klassifizierung durchzuführen, die sich nur auf die relevanten Interaktionsinformationen konzentriert. Zu diesem Zweck können der Beginn und die Verschiebung eines möglichen Interaktionsereignisses zum Beispiel mit Algorithmen zur Erkennung abrupter Veränderungen (z. B. Hypothesentests des Hinkley-Page-Tests) oder mit Algorithmen zur Erkennung von Ableitungen ermittelt werden.

Die so bearbeiteten Daten können zur Audifikation und/oder zur Klassifizierung übertragen werden. Für einige Anwendungen kann allein eine Audifikation als Feedback zur Verfügung gestellt werden und für einige andere Anwendungen ist nur eine Klassifizierung notwendig. Es gibt auch Anwendungen, die eine Integration von Klassifizierung und Audifikation von Interaktionsereignissen benötigen.

Die *Audifikation* kann in verschiedene Arten unterteilt werden. Bei der direkten Audifikation handelt es sich um eine direkte Umwandlung des VA-Signals in einen hörbaren Klangbereich unter Verwendung verschiedener Standardverarbeitungswerkzeuge wie Filterung, Verstärkungsoperatoren oder Effektgenerierung. Sie kann vier Hauptteilschritte umfassen, nämlich die Filterung des Signals zur Verbesserung der Frequenzkomponenten von Signalereignissen oder Signalsegmenten von Interesse, die mit den für ein bestimmtes Verfahren signifikanten Wechselwirkungen zwischen Instrumenten-Spitze/Schaft und Gewebe/Oberfläche zusammenhängen. Diese Filterstufe kann mit verschiedenen Techniken durchgeführt werden, beispielsweise mit der linearen Filterung, der adaptiven Filterung oder der Filterung, die mit einer beliebigen Art von Signalzerlegungstechniken erzeugt wird, wie beispielsweise der kontinuierlichen Wavelet-Transformation (CWT), der diskreten Wavelet-Transformation (DWT), der empirischen Modedekomposition (EMD) oder der autoregressiven Modellierung.

Das Signal wird in verschiedene Skalen, Modi oder Dynamiken zerlegt (je nach verwendeter Technik) und dann unter Verwendung von Skalen/Modi/Dynamiken rekonstruiert, in denen Interaktionsereignisse von Interesse vorhanden sind, dies können beispielsweise Interaktionsereignisse mit hoher Energie sein.

Die *Lautstärkeanpassung* besteht aus der Anwendung einer Verstärkung auf das VA-Signal, um die Lautstärke zu erhöhen. Sie kann auch zur Begrenzung zu lauter Ereignisse oder gesättigter Audiosignalsegmente verwendet werden.

Die *Dynamikbearbeitung* dient der Veränderung der dynamischen Eigenschaften des Audiosignals. Sie ermöglicht die Einstellung eines Verhältnisses zwischen den Eingangspegeln und den Ausgangspegeln. Dies kann in einem einzigen Frequenzband oder in mehreren Frequenzbändern für eine unabhängige, parallele Dynamikbearbeitung erfolgen.

Die *Erzeugung von Effekten* hat zum Ziel, den Klang des Audiosignals zu manipulieren. Übliche Effekte, die hier verwendet werden können, sind Nachhall, Hall, Faltung, Echo, Chorus, Zeitdehnung, Transposition, Harmonizer.

Die *indirekte Audifikation* umfasst die Verknüpfung von Merkmalen des VA-Signals mit auditiven Parametern zum Zweck der Datendarstellung. Dieser Schritt umfasst die Extraktion von Merkmalen, die dann in eine auditive Darstellung umgewandelt werden, indem die Merkmale verschiedenen Parametern des angezeigten Tons zugeordnet werden. Sie umfasst drei Hauptunterschritte, nämlich zunächst die

*Merkmalsextraktion,* hier werden verschiedene Merkmale oder Attribute aus dem Signal extrahiert. Es können verschiedene Arten von Merkmalen aus dem Signalsegment extrahiert werden, wie beispielsweise Merkmale im Zeitbereich, im Frequenzbereich oder im Zeit-Frequenz-Bereich. Beispiele für Merkmale im Zeitbereich sind Signalstärken oder -energien, statistische Merkmale wie Signalmittelwert oder -varianz und morphologische Merkmale. Für Merkmale im Frequenzbereich können Schätzungen des Signalspektrums mit verschiedenen Spektralschätzungsmethoden wie FFT, AR-Modellierung und anderen Methoden durchgeführt werden. Für Merkmale im Zeit-/Frequenzbereich können Methoden wie die Kurzzeit-Fourier-Transformation (STFT), das Mel-Frequenz-Cepstrum, die Wigner-Ville-Verteilung, die kontinuierliche Wavelet-Transformation (CWT), die diskrete Wavelet-Transformation (DWT), die empirische Modenzerlegung (EMD), die zeitvariable autoregressive Modellierung, Zyklostationaritätsmerkmale und andere Methoden verwendet werden.

Beim *Mapping* werden die Merkmale dann in Audioparameter umgewandelt, die zur Erzeugung einer auditiven Darstellung dienen. Dies kann geschehen durch eine direkte Zuordnung der extrahierten Merkmale über eine Parameter-Mapping-Sonifikation, bei der beispielsweise Änderungen der Signalstärke auf Änderungen der Tonhöhe der resultierenden akustischen Darstellung abgebildet werden können; durch ein dynamisches Modell, das die zeitliche Entwicklung eines Systems beschreibt, das durch die Merkmale angeregt wird, wobei das Modell eine physikalische Schallquelle simulieren soll; oder mit Hilfe von *Entscheidungsregeln, Clustermethoden* oder einem *trainierten maschinellen Lernmodell,* das die Merkmale als Eingaben verwendet und klassifizierte Ereignisse als Ausgaben erzeugt, die dann für die Parametrisierung des Geräuschs verwendet werden.

Die *Klangsynthese* dient der Erzeugung von bestimmten Klangeigenschaften.

Im Schritt der *Tonprojektion* wird alles Notwendige für die Übertragung der Daten und Anzeige der Tonausgabe an die Rückmelde-/Speichereinheit, über einen oder mehrere Lautsprecher oder Kopfhörer bereitgestellt.

Die *Klassifizierung* kann mehrere der folgenden Schritte umfassen.

Darunter zunächst die *Identifizierung transienter und stationärer Dynamiken:* Die aus den Wechselwirkungen zwischen medizinischem Instrument und Gewebe oder Oberfläche resultierenden VA-Signale weisen eine sehr komplexe Dynamik auf. Ein einziges Interaktionsereignis kann in einem sehr kurzen Zeitintervall eine Mischung aus transienter und stationärer Dynamik aufweisen. Zum Beispiel kann es bei einem Tastvorgang in dem Moment, in dem die Spitze des Instruments in Kontakt mit der VA-Anregung kommt, zu einer extrem kurzzeitigen transienten Reaktion kommen, gefolgt von einer stationären Reaktion, die sich aus dem effektiven Abtastvorgang ergibt. Inhomogenitäten der Oberfläche können zu Kollisionen der Spitze mit der Oberfläche führen, was wiederum zu einer Mischung aus transienter und stationärer Dynamik führt. In dem Moment, in dem die Palpation aufhört, kann auch der Kontaktstopp eine transiente Dynamik erzeugen, so dass in einem kurzen Zeitintervall eine Reihe von transienten-stationären Dynamiken auftreten können. Diese Komplexität führt dazu, dass das Signal nicht mit Standard- oder klassischen Signalverarbeitungsalgorithmen verarbeitet werden kann, die normalerweise für Sprache oder Musiktöne verwendet werden, deren akustische Antworten in einem kurz- bis mittelfristigen Zeitintervall recht stabil sein können.

Bei der *Spektralbandzerlegung* wird das VA-Signal in mehrere Schmalbandsignale zerlegt, indem das Gesamtfrequenzband des Signals in mehrere Teilfrequenzbänder zerlegt wird. Das Hauptziel besteht darin, später verschiedene Aspekte des Signals getrennt zu verarbeiten. Wir gehen davon aus, dass sich eine Signal-Charakteristik aus mehreren Dynamiken zusammensetzt, die Unregelmäßigkeitseigenschaften der Textur wie Glätte oder Rauheit repräsentieren. Daher können die Signale in mehrere Dynamiken zerlegt werden, die z. B. die Stufen der Unregelmäßigkeiten in der Interaktion darstellen können. Die Anzahl der Frequenzbänder, in die das Signal zerlegt wird, hängt davon ab, ob das Signal durch Einführen, Abtasten oder eine andere Art der Interaktion zwischen Instrument und Gewebe/Oberfläche entsteht. Die Anzahl der Bänder kann zwischen 2 und 10 Frequenzteilbändern variieren. Die Zerlegung kann mit verschiedenen Methoden durchgeführt werden, z. B. Filterbanken, kontinuierliche Wavelet-Transformation (CWT), diskrete Wavelet-Transformation (DWT), Polverfolgung und zeitvariante autoregressive Modellierung, empirische Modedekomposition (EMD) usw.

Sowohl die zuvor beschriebene *Identifizierung transienter und stationärer Dynamiken* als auch die *Spektralbandzerlegung* und ihrer Kombination sind neue und erfinderische Verarbeitungsverfahren, die in der Literatur nicht bekannt sind. Wie bereits erwähnt, enthält das VA-Signal viele verschiedene Dynamiken im Signal, die sich nur sehr schwer analysieren oder zusammen verarbeiten lassen. Es ist notwendig, die Komplexität des VA-Signals zu verringern, bevor die Phase der Extraktion von Indikatoren und Merkmalen beginnt. Wenn Signale in verschiedene Aspekte getrennt werden, wie beispielsweise bei der *Identifizierung transienter und stationärer Dynamiken* in transiente und oszillatorische Merkmale und/oder bei der *Spektralbandzerlegung* in verschiedene Bandbreiten und/oder der Demodulation des Signals in verschiedenen Modi, können Merkmale besser mit den extrahierten Aspekten in Verbindung gebracht werden.

Da Interaktionen in hohem Maße mit abrupten Veränderungen einhergehen können (Kontakt mit Gewebe, Gewebeumklammerung, Einstich und so weiter), sollten zur Bewertung dieser Veränderungen Detektoren für abrupte Veränderungen verwendet werden, um den gesamten Umfang dieser Veränderungen berücksichtigen zu können.

*Berechnung von Indikatoren:* Vor dem Schritt der Merkmalsextraktion ist es notwendig, zunächst Indikatoren aus den Signalen zu extrahieren. Diese Indikatorberechnung erhält als Eingabe normalerweise ein oder mehrere Signale und liefert als Ausgabe ein oder mehrere Signale oder Matrizen. In der vorliegenden Signalverarbeitungsmethodik finden drei Arten von Indikatoren Anwendung, wie als erstes *Zeitbereichsindikatoren,* also Indikatoren, die direkt aus den Zeitreihen berechnet werden.

Es können drei Arten von *Zeitbereichsindikatoren* berechnet werden, nämlich *statistische Indikatoren:* Indikatoren, die durch statistische Standardoperationen berechnet werden, beispielsweise durch Schätzung der Wahrscheinlichkeitsverteilung (datenbasiert oder modellbasiert) oder durch Berechnung des zeitabhängigen Durchschnitts oder der Varianz, zum Beispiel unter Verwendung eines zeitlich gleitenden Fensters. *Morphologische Indikatoren:* Indikatoren, die verschiedene Aspekte der Morphologie des Signals betonen. Beispiele für diese Art von Indikatoren sind Ableitungen, Krümmungsindikatoren oder Ableitungsindikatoren zweiten Grades, Polynomanpassung, Schätzung des Überschwingens. Auch die Darstellung in Form der bekannten ADSR-Hüllkurve kann als morphologischer Indikator verwendet werden. Auf *plötzlichen Änderungen basierende Indikatoren:* Indikatoren, die Änderungen im Signal bewerten, die in einem kurzen Zeitintervall auftreten. Zum Beispiel kann in dem Moment, in dem das Instrument mit einer Oberfläche in Kontakt kommt, die abrupte Änderung des Signals Informationen über die Härte der Oberfläche liefern. Ein anderes Beispiel ist der Einstich einer Nadel in eine Schicht, bei dem die abrupte Änderung, die sich aus dem Bruch des Gewebes ergibt, mit der Elastizität des Gewebes und seiner Widerstandseigenschaft gegenüber der Instrumentenspitze in Verbindung gebracht werden kann. Die abrupte Veränderung kann beispielsweise mit Hilfe eines statistischen Hypothesentests wie dem Hinkley- und Page-Test oder dem CUSUM-Test bewertet oder berechnet werden.

*Indikatoren im Frequenz- und Zeit-Frequenz-Bereich* wie *spektrumbasierte Indikatoren,* die dem stationären Spektrum (beispielsweise ein Periodogramm) oder dem Zeit-Frequenz-Spektrum entsprechen (z. B. unter Verwendung von Spektrogramm, Wygner-Ville, CWT, zeitvarianter autoregressiver Modellierung, usw.). *Indikatoren auf der Grundlage der momentanen dominanten Frequenz,* die aus dem Zeit-Frequenz-Spektrum als augenblickliche dominante Frequenz berechnet werden, d. h. die Frequenz, die zu jedem Zeitpunkt die maximale Energie im Zeit-Frequenz-Spektrum enthält. Diese kann direkt aus der Zeit-Frequenz-Matrix oder parametrisch über ein Zeitvariantenmodell wie bei TVAR unter Verwendung von Polschätzung/- verfolgung berechnet werden.

Das Konzept der *dominanten Frequenz* wird in der Literatur lediglich für stationäre Spektralanalysen (beispielsweise auf der Grundlage der FFT) verwendet, bei denen die Zeit nicht berücksichtigt werden muss. In der Musik- und Sprachsignalverarbeitung ist ein ähnliches Konzept die Tonhöhe. Die Tonhöhe bezieht sich jedoch auf eine "Grundperiode" des Signals, und diese bezeichnet Signale, die recht stationär sind (die spektralen Eigenschaften ändern sich nicht drastisch mit der Zeit). Im vorliegenden Fall wird aufgrund der ausgeprägten nicht-stationarität der Signale zu jedem Zeitpunkt eine Schätzung der Frequenzkomponente durchgeführt, die gegenüber den anderen dominiert. Durch diese Erweiterung des Konzepts zur *momentanen dominanten Frequenz,* können damit Dynamiken und damit Informationen aus transienten oder hochgradig nicht-stationären Signalen gewonnen werden.

Der Verfahrensschritt, der für die Berechnung der *momentanen dominanten Frequenz* beispielsweise im Beispiel der Palpation vorgeschlagen wird, ist im Stand der Technik nicht bekannt.

Schließlich sind *hüllkurvenbasierte Indikatoren* extrahierte Indikatoren, die der Hüllkurve des Signals entsprechen. Die Hüllkurve kann beispielsweise mittels DWT oder Hilbert Transformation mit oder ohne homomorphe Filterung berechnet werden.

*Merkmalsberechnung:* Es können vier Merkmalsfamilien extrahiert werden, die in insgesamt zehn Unterfamilien unterteilt sind. Jedes Merkmal entspricht einer skalaren Zahl.

Zur *Merkmalsberechnung* gehören *Zeitbereichsmerkmale* erhalten als Eingabe einen Zeitbereichsindikator und dienen zur Berechnung verschiedener Familien von Merkmals-Skalarwerten. Darunter fallen *statistische Merkmale im Zeitbereich* zur Berechnung klassischer statistischer Werte aus einem Indikator, beispielsweise Durchschnittswerte, Varianz, Kurtosis, Schiefe usw. Darunter fallen weiterhin *morphologische Merkmale im Zeitbereich* zur Berechnung morphologischer Werte aus den Indikatoren wie Steigungen, maximale Steigungen, Krümmungen, Latenzen, Dauer der Segmente und Überschwinger. Das Hauptziel dieser Familie von Merkmalen ist die Bewertung der Morphologie der VA-Wellenerregung im Zeitverlauf, die aus einem Interaktionsereignis resultiert. Hierzu gehören weiterhin *auf abrupten Veränderungen basierende Merkmale im Zeitbereich,* wobei diese Merkmalsfamilie darauf abzielt, die Größe der Veränderung zu Beginn oder am Ende eines Interaktionsereignisses oder einige abrupte Veränderungen zu quantifizieren, die während eines Interaktionsereignisses aufgrund von Inhomogenitäten im Gewebe/an der Oberfläche auftreten. Höhere Werte dieser Indikatoren könnten auf härteres Gewebe hindeuten und andersherum.

Zur *Merkmalsberechnung* gehören *Spektralbereichs-Merkmale* zur Berechnung von Merkmals-Skalarwerten aus dem stationären Spektrum (Vektor) oder aus dem Zeit-Frequenz-Spektrum (Matrix). Hierzu gehören *Zeit-Frequenz-Spektrum-Ereignis-Merkmale,* also Werte im Zusammenhang mit Ereignissen, die im Zeit-Frequenz-Spektrum identifiziert werden können. Beispiele für Ereignisse sind maximale Spektralenergien, abrupte Änderungen der Spektralenergien, abrupter Energieübergang von einer Frequenz zur anderen, Wechsel von nicht stationären zu stationären Frequenzkomponenten usw. Hierzu gehören weiterhin *Spektralenergiemerkmale,* also Merkmale, die sich auf die Energien im Spektrum beziehen. Sie können als absolute oder relative Energien berechnet werden. Die relativen Energien können Verhältnisse zwischen den Energien in verschiedenen Bändern sein (beispielsweise berechnet aus der *Spektralbandzerlegung)* oder relativ zur Gesamtenergie des Signals sein. Diese Familie von Merkmalen gibt Aufschluss über die spektrale Verteilung der an der Wechselwirkung beteiligten spektralen Energien. Wenn beispielsweise eine Nadel eine Gewebeschicht durchbricht, werden im Allgemeinen hochfrequente Komponenten angeregt. Daher werden in diesem Fall die Energieverhältnisse im Hochfrequenzbereich hervorgehoben. Bei der Palpation sind hohe Frequenzen charakteristisch für Wechselwirkungen mit hartem Gewebe und niedrige Frequenzen für Wechselwirkungen des Werkzeugs mit weichem Gewebe. Hierzu gehören weiterhin *statistische Merkmale der momentanen dominanten Frequenz* zur Berechnung klassischer statistischer Indikatoren aus dem Indikator der momentanen dominanten Frequenz, wie Mittelwerte und Varianz. *Statistische Werte der momentanen dominanten Frequenz* können direkt mit der Homogenität der interagierenden Oberfläche oder des Gewebes in Verbindung gebracht werden. Eine hohe Varianz der momentanen dominanten Frequenz zeigt beispielsweise an, dass das interagierende Gewebe sehr inhomogen ist, und eine geringe Varianz kann ein Merkmal eines glatten Gewebes oder eines Gewebes sein, dessen Eigenschaften sich im Raum nicht wesentlich ändern. Hierzu gehören weiterhin *dynamische Merkmale der momentanen dominanten Frequenz,* also Merkmale, die darauf abzielen, das zeitliche Verhalten der momentanen dominanten Frequenz zu beschreiben. Eine dominante Frequenz kann in der Zeitdomäne oder in ihrer 2d komplexen Darstellung verfolgt werden. Im Zeitbereich wirkt die dominante Frequenz wie ein Signal, dessen Dynamik (oszillierend, stationär, transient usw.) als Wert extrahiert werden kann. In der 2d komplexen Darstellung kann eine dominante Frequenz wie ein Teilchen fungieren, das sich in einem 2d-Raum bewegt. Dieses Teilchen beinhaltet eine Bewegung in der Zeit, die quantifiziert und in Merkmalswerte umgewandelt werden kann.

Zur *Merkmalsberechnung* gehören weiterhin *Hüllkurvenbasierte Merkmale,* also Merkmale, die direkt aus der Hüllkurve im Zeitbereich oder aus einer spektralen Transformation der Hüllkurve berechnet werden. Manchmal handelt es sich hierbei um zwei komplexe Interaktionen, die ein hohes Maß an nichtlinearer Dynamik aufweisen und stark moduliert sind (z. B. Bohren oder Abtasten stark inhomogener Strukturen), die mit Hilfe der Hüllkurve des Signals vereinfacht werden können. Hierzu zählen *spektrale Hüllkurvenmerkmale,* also Merkmale, die aus dem stationären oder zeitlichen Frequenzspektrum der Hüllkurve berechnet werden. Hierzu zählen auch *hüllkurvenbasierte Merkmale im Zeitbereich,* also Merkmale, die direkt aus dem Hüllkurvenindikator berechnet werden. Sie können wichtige Informationen über Dämpfung und Unregelmäßigkeiten im interagierenden Gewebe liefern. So kann beispielsweise die Anzahl der Spitzen und Täler der Hüllkurve Aufschluss über Unebenheiten geben, die durch die Interaktion zwischen Werkzeug und Gewebe/Oberfläche entstehen. Diese Unebenheiten können mit starken Unregelmäßigkeiten zusammenhängen, die dem Gewebe innewohnen können oder, im Falle der Palpation, mit darunter liegenden Strukturen zusammenhängen.

Zur *Merkmalsberechnung* gehören auch *Deep-Learning-basierte Merkmale.* Deep-Learning-basierte Merkmale werden von einem mehrschichtigen neuronalen Netz berechnet, das die Daten auf nichtlineare Weise verarbeitet. Zu diesem Zweck können die Daten der *Spektralbandzerlegung* oder die der *spektrumbasierten Indikatoren* als Eingangsdaten in den vorgesehenen Deep-Learning-Algorithmus eingespeist werden, z. B. in mehrschichtige Perzeptronen (MLPs), Faltungsneuronale Netze (CNN), rekurrente neuronale Netze (RNNs), Langzeitspeichernetze (LSTMs), Autoencoder und eingeschränkte Boltzmann-Maschinen (RBM). Jeder dieser Algorithmen kann auf der Grundlage der Charakterisierung der Eingabedaten automatisch eine Reihe von Merkmalen auf höherer Ebene erlernen.

Die extrahierten Merkmale können in einen Vektor eingeordnet werden, der in ein *trainiertes Modell des maschinellen Lernens* eingespeist wird, das mit verschiedenen Architekturen wie Support Vector Machine (SVM), k-Nearest Neighbors (kNN), Random Forest, neuronalen Netzen und vielen anderen Ansätzen erstellt werden kann. Das trainierte Machine-Learning-Modell ordnet die Interaktionsereignisse des Eingangssignals VA vordefinierten Klassen zu. Beispiele für Klassen sind: Bei Palpationsereignissen sind dies beispielsweise verschiedene Klassen von Weichheit und Härte oder Rauheit und Glätte des Gewebes. Bei Insertionsereignissen sind dies verschiedene Klassen von Insertionsgeweben (beispielsweise Fettgewebe, Muskelgewebe oder Bindegewebe) oder verschiedene Klassen von Schichten wie Faszien, Peritoneum usw. Bei laparoskopischen Eingriffen könnten die Klassen beispielsweise für verschiedene Aufgaben (Greifen, Schneiden, Abtasten, Nähen usw.) angegeben werden oder die Klassen könnten die verschiedenen Instrumente darstellen, die erkannt werden können, wenn sie mit dem Gewebe/der Oberfläche in Berührung kommen (beispielsweise Greifer, Pinzette oder Schere).

Schließlich könnte die Identifizierung der verschiedenen Klassen in einem letzten Schritt für verschiedene Zwecke wie die Überwachung, Unterstützung, Speicherung, um nur einige zu nennen, gespeichert oder angezeigt werden.

### Spezifische Verarbeitungsbeispiele

Das allgemein beschriebene Verfahren kann für spezifische Anwendungen durch die Anpassung einzelner Verfahrensschritte optimiert werden, beispielsweise zur Klassifizierung der Gewebehärte/-weichheit bei der Palpation. Der grundlegende Erfindungsgedanke besteht darin, Signalmerkmale mit Gewebe-/Oberflächenweichheits-/Härteklassen zu verknüpfen. So ist es beispielsweise möglich, die GewebeweichheitZ-härte in zehn Klassen zu unterteilen, von Klasse 1 für sehr weiches Gewebe bis Klasse 10 für sehr hartes Gewebe. Ein Algorithmus für maschinelles Lernen, der mit Geweben trainiert wurde, deren Weichheits-/Härtegrad bekannt ist, kann den definierten Klassen Signalmerkmale zuordnen, was zu einem durch maschinelles Lernen trainierten Modell führt, das in der Lage ist, ein Palpationsereignis automatisch in die verschiedenen Weichheits-/Härteklassen einzuordnen.

Spezifische Verarbeitungsbeispiele sind Gegenstand der nachfolgenden Figuren.

Die erfindungsgemäße Vorrichtung und das erfindungsgemäße Verfahren weisen gegenüber dem Stand der Technik eine ganze Reihe von Vorteilen auf. Die Erfassung und erfindungsgemäße Verarbeitung vibroakustischer Signale ermöglichen es, eine Intervention wesentlich detailreicher und mit höherer Dynamik zu erfassen, da vibroakustische Signale nicht den Beschränkungen beispielsweise visueller Verfahren unterliegen. Die vibroakustischen Wellen breiten sich auch außerhalb des Sichtfeldes oder der Sichtlinie des Anwenders aus. Zudem sind bei der erfindungsgemäßen Vorrichtung keine aktiven Komponenten im Sinne einer Ultraschallsonde notwendig, die also zumindst einen Teil zu erfassenden Signals zunächst selbst erzeugen. Die erfindungsgemäße Vorrichtung funktioniert rein passiv, nimmt also nur Signale auf, die durch die Interaktion entstehen. Ebenso sind die erfassten Daten wesentlich weniger speicherintensiv und können entsprechend problemloser archiviert werden.

Die Erfindung sowie das technische Umfeld werden nachfolgend anhand der Figuren näher erläutert. Es ist darauf hinzuweisen, dass die Figuren eine besonders bevorzugte Ausführungsvariante der Erfindung zeigen. Die Erfindung ist jedoch nicht auf die gezeigte Ausführungsvariante beschränkt. Insbesondere umfasst die Erfindung, soweit es technisch sinnvoll ist, beliebige Kombinationen der technischen Merkmale, die in den Ansprüchen aufgeführt oder in der Beschreibung als erfindungsrelevant beschrieben sind.

Es zeigen:
- Fig. 1: eine schematische Ansicht des generellen Aufbaus der Vorrichtung;
- Fig. 2: die erste Ausführungsform der Vorrichtung speziell zur Verwendung mit einer Hakensonde;
- Fig. 3: die zweite Ausführungsform der Vorrichtung speziell zur Verwendung bei der Palpation;
- Fig. 4: die dritte Ausführungsform der Vorrichtung speziell zur Verwendung mit einer Veres-Nadel;
- Fig. 5: die vierte Ausführungsform der Vorrichtung speziell zur Verwendung mit einer Biopsie-Nadel;
- Fig. 6: die fünfte Ausführungsform der Vorrichtung speziell zur Verwendung mit einem Führungsdraht eines Kathetersystems;
- Fig. 7: die sechste Ausführungsform der Vorrichtung speziell zur Verwendung mit einem laparoskopischen Instrument bei einer Roboter assistierten Intervention (RAS);
- Fig. 8: die siebte Ausführungsform der Vorrichtung speziell zur Verwendung mit einem stabförmigen laparoskopischen Instrument;
- Fig. 9: Beispiel der Anwendung der erfindungsgemäßen Vorrichtung bei der Palpation;
- Fig. 10: Beispiel der Anwendung der erfindungsgemäßen Vorrichtung bei der Einführung einer Punktionsnadel;
- Fig. 11: Beispiel der Anwendung mehrerer erfindungsgemäßer Vorrichtungen bei einem laparoskopischen Eingriff;
- Fig. 11A: Beispiel der Anwendung der erfindungsgemäßen Vorrichtung beim chirurgischen Bohren;
- Fig. 11B: Beispiel der Anwendung der erfindungsgemäßen Vorrichtung zur Erkennung von Kollisionen bei Roboter assistierten Eingriffen;
- Fig. 12: Beispiel einer Anwendung der erfindungsgemäßen Vorrichtung bei einem laparoskopischen Eingriff zur Erkennung von Interaktionen außerhalb des Sichtfeldes (FOV) des endoskopischen Kamerasystems;
- Fig. 13: Beispiel einer Anwendung der erfindungsgemäßen Vorrichtung bei einem laparoskopischen Eingriff zur Erkennung von Interaktionen außerhalb der Sichtlinie (LOS) des endoskopischen Kamerasystems;
- Fig. 14: das Flussdiagramm der allgemeinen Signalverarbeitungsmethodik beziehungsweise der Signal- oder Datenverarbeitung;
- Fig. 15: das Verarbeitungsflussdiagramm für eine spezifische Anwendung zur Klassifizierung der Gewebehärte/-weichheit bei der Palpation;
- Fig. 16: das Verarbeitungsflussdiagramm für eine spezifische Anwendung zur indirekten Prüfung einer Nadelpunktion;
- Fig. 17: das Verarbeitungsflussdiagramm für eine spezifische Anwendung zur automatischen Erkennung von laparoskopischen Werkzeugen, wenn diese durch den Trokar in den chirurgischen Eingriff gelangen;
- Fig. 18: das Verarbeitungsflussdiagramm für eine spezifische Anwendung zur Evaluierung von laparoskopischen Eingriffen, bei der jede während des Eingriffs auftretende Interaktion zwischen Werkzeug und Gewebe monitoriert und bewertet wird.
- Fig. 19: das Verarbeitungsflussdiagramm für eine spezifische Anwendung zur vibroakustischen Echtzeit-Überwachung während elektrochirurgischer Eingriffe zur Charakterisierung von Gewebe.
- Fig. 20: das Verarbeitungsflussdiagramm für eine spezifische Anwendung zur vibroakustischen Echtzeit-Überwachung für präzise Knochenbohrungen bei chirurgischen Eingriffen.
- Fig. 21: das Verarbeitungsflussdiagramm für eine spezifische Anwendung zur Erkennung und Vorbeugung von Schäden an pulsierenden Strukturen in der laparoskopischen und robotergestützten Chirurgie.
- Fig. 22: das Verarbeitungsflussdiagramm für eine spezifische Anwendung zur Echtzeit-Erkennung und Klassifizierung von Kollisionen mit der erfindungsgemäßen Technologie.

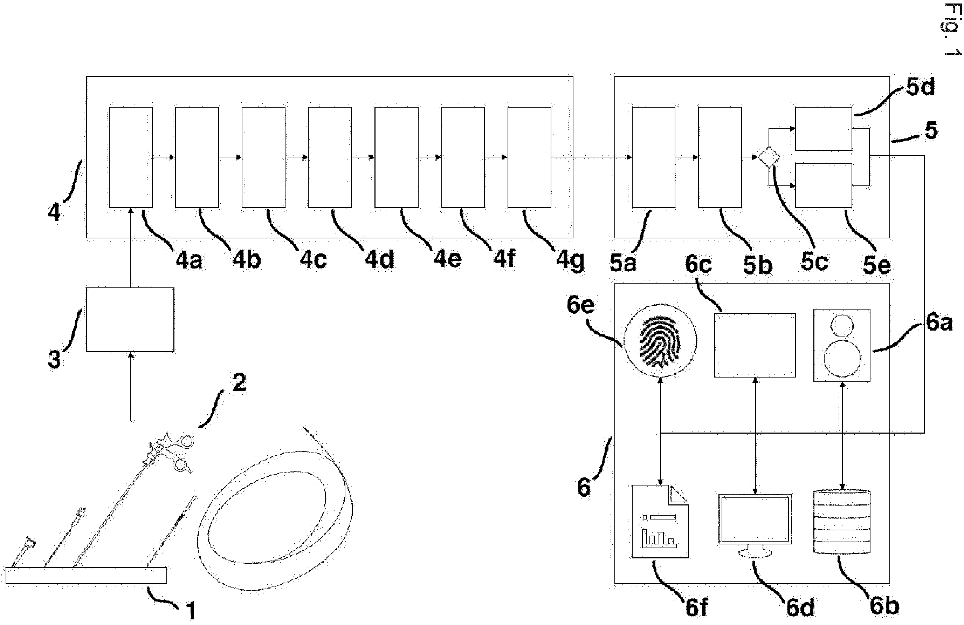

Figur 1 zeigt eine schematische Darstellung der erfindungsgemäßen Vorrichtung zur Erfassung von vibroakustischen Signalen und des entsprechenden Verfahrens zur Auswertung von vibroakustischen Signalen.

Die erfindungsgemäße Vorrichtung umfasst eine Signalerfassungsvorrichtung 4 mit einem Kopplungselement 4a zum Festlegen zumindest der Signalerfassungsvorrichtung 4 beispielsweise am proximalen Ende eines medizinischen Instruments 2. An das Kopplungselement 4a kann ein mechanischer Verstärkungsmechanismus 4b beispielsweise in Form einer Membran und/oder eines Schallraumes vorgesehen sein. An das Kopplungselement 4a oder an den mechanischen Verstärkungsmechanismus 4b schließt sich ein Akustik- beziehungsweise Vibrationssensor 4c an, der geeignet ist, vibroakustische Signale zu erfassen. Die vom Sensor 4c erfassten Signale werden optional zunächst auf analoger Ebene verarbeitet 4d und anschließend einem Analog-Digital-Wandler 4e zugeführt. Ein Controller 4f steuert die Vorgänge und eine Kommunikationseinheit 4g regelt die kabellose oder kabelgebundene Übertragung der digitalen Daten an die Daten-/Signalverarbeitungsvorrichtung 5.

Die Daten-/Signalverarbeitungsvorrichtung 5 ist vorgesehen, das Programm für das erfindungsgemäße computerimplementierte Verfahren zur Verarbeitung der vibroakustischen Signale beziehungsweise der entsprechenden digitalen Daten auszuführen, die die Signalerfassungsvorrichtung 4 an die Daten-/Signalverarbeitungsvorrichtung 5 übermittelt hat. Das Verfahren umfasst die Schritte der digitalen Signalvorverarbeitung 5a, der Identifizierung von Segmenten und Regionen von Interesse 5b und eine sich anschließende Audifikation 5d und/oder 5c Klassifizierung 5e. Die spezifischen Unterschritte des Verfahrens sind in Figur 14 dargestellt und können insgesamt oder teilweise Gegenstand des Verfahrens sein. Spezifische Verfahrensabläufe sind Gegenstand einiger der folgenden Figuren.

Die Ergebnisse des Verfahrens bezüglich der Audifikation 5d und/oder Klassifizierung 5e werden kabellos oder kabelgebunden an die Ausgabevorrichtung 6 übertragen. Die Ausgabevorrichtung 6 wandelt das Ergebnis entsprechend den Anforderungen entweder über einen Lautsprecher 6a in ein akustisches Signal um oder speichert das Ergebnis gegebenenfalls auch in einer Datenbank 6b, gibt das Ergebnis an ein Virtual Reality (VR) oder Augmented Reality (AR) Gerät 6c aus oder visualisiert das Ergebnis auf einem Bildschirm 6d. Ebenso ist die Ausgabe des Ergebnisses über ein haptisches Gerät 6e, beispielsweise einen Vibrationsmechanismus, oder direkt als Ausgabe in Berichtsform 6f denkbar.

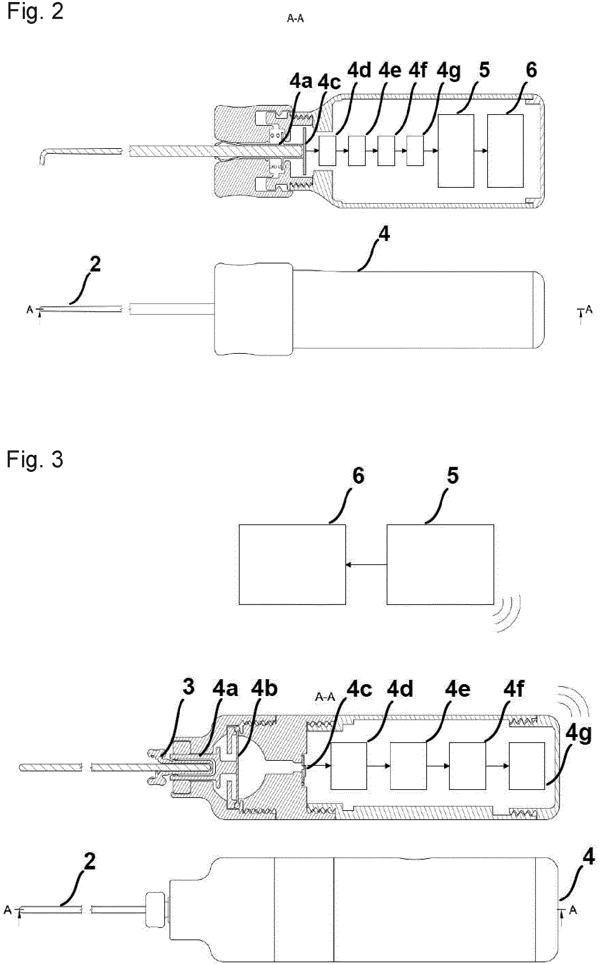

Figur 2 zeigt eine erste Ausführungsform der erfindungsgemäßen Vorrichtung speziell zur Verwendung mit einer Hakensonde. Die Vorrichtung umfasst in dieser Ausführungsform eine Signalerfassungsvorrichtung 4 mit einem Kopplungselement 4a zum Festlegen zumindest der Signalerfassungsvorrichtung 4 am proximalen Ende eines medizinischen Instruments 2, nämlich einer Hakensonde. Das Kopplungselement 4a ist bei dieser Ausführungsform mit seinem proximalen Ende direkt mit dem mindestens einen Sensor verbunden und geeignet, vibroakustische Signale möglichst störungs- und verlustfrei von dem Instrument 2 an den mindestens einen Sensor 4c zu übertragen.

Bei einer direkten Verbindung ist der mindestens eine Sensor 4c direkt mit dem proximalen Ende des Kopplungselements 4a verbunden, wobei der Sensor 4c dann vorzugsweise ein Piezo- oder Kristallmikrofon ist.

Die vom Sensor 4c erfassten Signale werden optional zunächst auf analoger Ebene verarbeitet 4d und anschließend einem Analog-Digital-Wandler 4e zugeführt. Ein Controller 4f steuert die Vorgänge und eine Kommunikationseinheit 4g regelt die kabellose oder kabelgebundene Übertragung der digitalen Daten an die Daten-/Signalverarbeitungsvorrichtung 5. Von der Daten-/Signalverarbeitungsvorrichtung 5 werden die Ergebnisse an die Ausgabevorrichtung 6 übertragen.

Bei dieser Ausführungsform sind die wesentlichen Elemente der erfindungsgemäßen Vorrichtung, nämlich die Signalerfassungsvorrichtung 4, die Daten-/Signalverarbeitungsvorrichtung 5 und die Ausgabevorrichtung 6 vorzugsweise in einem gemeinsamen Gehäuse untergebracht.

Figur 3 zeigt eine zweite Ausführungsform der erfindungsgemäßen Vorrichtung speziell zur Verwendung bei der Palpation. Die erfindungsgemäße Vorrichtung umfasst in dieser Ausführungsform eine Signalerfassungsvorrichtung 4 mit einem Kopplungselement 4a zum Festlegen zumindest der Signalerfassungsvorrichtung 4 am proximalen Ende eines medizinischen Instruments 2, nämlich eines Taststabs. Das Kopplungselement 4a ist mit seinem proximalen Ende indirekt mit dem mindestens einen Sensor 4c verbunden und geeignet, vibroakustische Signale möglichst störungs- und verlustfrei von dem Instrument 2 an den mindestens einen Sensor 4c zu übertragen.

Bei dieser indirekten Verbindung ist das proximale Ende des Kopplungselements 4a nicht direkt mit einem Sensor 4c, sondern vorzugsweise mit einer Membran 4b verbunden, die die vibroakustischen Signale in einen sich an die Membran 4b anschließenden Schallraum überträgt. Die Membran 4b ist geeignet, die vom Kopplungselement 4a übertragenen vibroakustischen Signale zu verstärken, wobei der Schallraum zu einer zusätzlichen Verstärkung des Signals führt. Grundsätzlich ist es auch denkbar, dass die vibroakustischen Signale direkt, also ohne eine zwischengeschaltete Membran 4b, vom Kopplungselement 4a in den Schallraum übertragen werden.

Bei einer indirekten Verbindung ist der mindestens eine Sensor 4c vorzugsweise ein Luftmikrofon.

Die vom Sensor 4c erfassten Signale werden optional zunächst auf analoger Ebene verarbeitet 4d und anschließend einem Analog-Digital-Wandler 4e zugeführt. Ein Controller 4f steuert die Vorgänge und eine Kommunikationseinheit 4g regelt die kabellose oder kabelgebundene Übertragung der digitalen Daten an die Daten-/Signalverarbeitungsvorrichtung 5. Von der Daten-/Signalverarbeitungsvorrichtung 5 werden die Ergebnisse an die Ausgabevorrichtung 6 übertragen.

Bei dieser Ausführungsform ist die Signalerfassungsvorrichtung 4 vorzugsweise getrennt von der Daten-/Signalverarbeitungsvorrichtung 5 und der Ausgabevorrichtung 6 untergebracht.

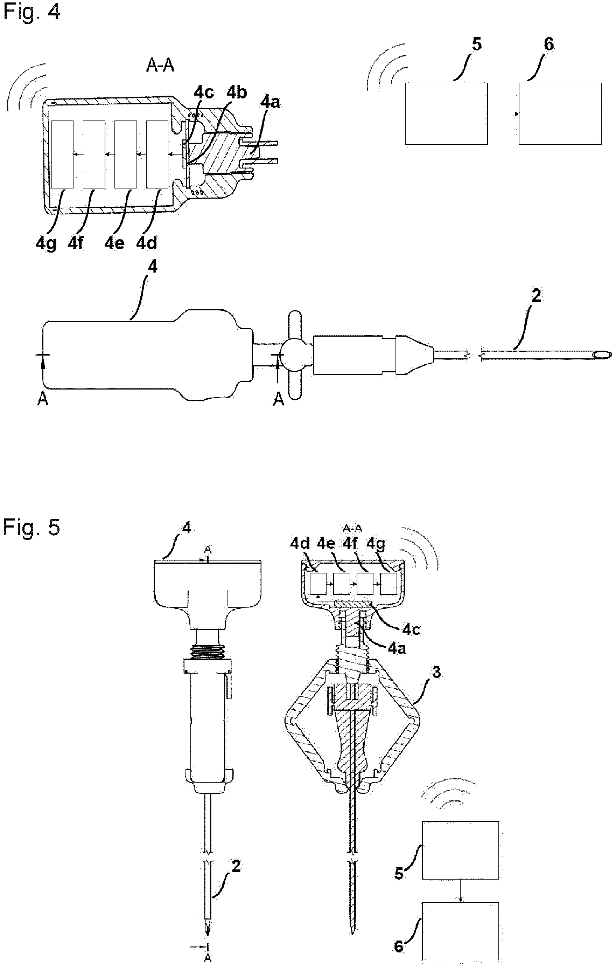

Figur 4 zeigt eine dritte Ausführungsform der erfindungsgemäßen Vorrichtung speziell zur Verwendung mit einer Veres-Nadel. Die erfindungsgemäße Vorrichtung umfasst in dieser Ausführungsform eine Signalerfassungsvorrichtung 4 mit einem Kopplungselement 4a zum Festlegen zumindest der Signalerfassungsvorrichtung 4 am proximalen Ende eines medizinischen Instruments 2, nämlich einer Veres-Nadel. Das Kopplungselement 4a ist mit seinem proximalen Ende indirekt mit dem mindestens einen Sensor4cverbunden und geeignet, vibroakustische Signale möglichst störungs- und verlustfrei von dem Instrument 2 an den mindestens einen Sensor 4c zu übertragen. Bei dieser indirekten Verbindung ist das proximale Ende des Kopplungselements 4a nicht direkt mit einem Sensor 4c, sondern vorzugsweise mit einer Membran 4b verbunden, die die vibroakustischen Signale verstärkt an einen Sensor 4c weiterleitet. Die Membran 4b ist geeignet, die vom Kopplungselement 4a übertragenen vibroakustischen Signale zu verstärken.

Bei dieser indirekten Verbindung ist der mindestens eine Sensor 4c vorzugsweise ein Piezo- oder Kristallmikrofon.

Die vom Sensor 4c erfassten Signale werden optional zunächst auf analoger Ebene verarbeitet 4d und anschließend einem Analog-Digital-Wandler 4e zugeführt. Ein Controller 4f steuert die Vorgänge und eine Kommunikationseinheit 4g regelt die kabellose oder kabelgebundene Übertragung der digitalen Daten an die Daten-/Signalverarbeitungsvorrichtung 5. Von der Daten-/Signalverarbeitungsvorrichtung 5 werden die Ergebnisse an die Ausgabevorrichtung 6 übertragen.

Bei dieser Ausführungsform ist die Signalerfassungsvorrichtung 4 vorzugsweise getrennt von der Daten-/Signalverarbeitungsvorrichtung 5 und der Ausgabevorrichtung 6 untergebracht.

Figur 5 zeigt eine vierte Ausführungsform der erfindungsgemäßen Vorrichtung speziell zur Verwendung mit einer Biopsie-Nadel. Die Vorrichtung umfasst in dieser Ausführungsform eine Signalerfassungsvorrichtung 4 mit einem Kopplungselement 4a zum Festlegen zumindest der Signalerfassungsvorrichtung 4 am proximalen Ende eines medizinischen Instruments 2, nämlich einer Biopsie-Nadel. Das Kopplungselement 4a ist bei dieser Ausführungsform mit seinem proximalen Ende direkt mit dem mindestens einen Sensor verbunden und geeignet, vibroakustische Signale möglichst störungs- und verlustfrei von dem Instrument 2 an den mindestens einen Sensor 4c zu übertragen.

Die Biopsie-Nadel 2 ist bei dieser vierten Ausführungsform in einem speziellen Adapterelement 3 angeordnet, um bestmöglich über das Kopplungselement 4a mit der Signalerfassungsvorrichtung verbunden zu sein. Das Adapterelement 3 umfasst zwei gewinkelte Elemente, die sich proximal und distal berühren und distal die Biopsie-Nadel umschließen und proximal zur Anbringung an dem Kopplungselement 4a vorgesehen sind.

Bei der direkten Verbindung ist der mindestens eine Sensor 4c direkt mit dem proximalen Ende des Kopplungselements 4a verbunden, wobei der Sensor 4c dann vorzugsweise ein Piezo- oder Kristallmikrofon ist.

Die vom Sensor 4c erfassten Signale werden optional zunächst auf analoger Ebene verarbeitet 4d und anschließend einem Analog-Digital-Wandler 4e zugeführt. Ein Controller 4f steuert die Vorgänge und eine Kommunikationseinheit 4g regelt die kabellose oder kabelgebundene Übertragung der digitalen Daten an die Daten-/Signalverarbeitungsvorrichtung 5. Von der Daten-/Signalverarbeitungsvorrichtung 5 werden die Ergebnisse an die Ausgabevorrichtung 6 übertragen.

Bei dieser Ausführungsform ist die Signalerfassungsvorrichtung 4 vorzugsweise getrennt von der Daten-/Signalverarbeitungsvorrichtung 5 und der Ausgabevorrichtung 6 untergebracht.

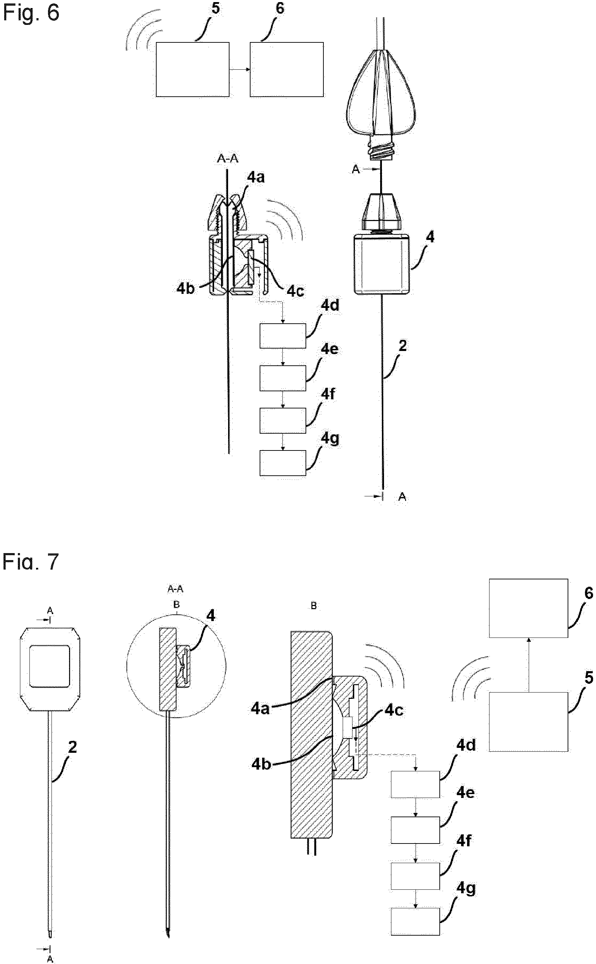

Figur 6 zeigt eine fünfte Ausführungsform der erfindungsgemäßen Vorrichtung speziell zur Verwendung mit dem Führungsdraht eines Kathetersystems. Die Vorrichtung umfasst in dieser Ausführungsform eine Signalerfassungsvorrichtung 4 mit einem Kopplungselement 4a zum Festlegen zumindest der Signalerfassungsvorrichtung 4 am proximalen Ende eines medizinischen Instruments 2, nämlich des Führungsdrahts eines Kathetersystems. Das Kopplungselement 4a durchläuft die Signalerfassungsvorrichtung 4 bei dieser Ausführungsform von proximal nach distal und bildet so einen Kanal, durch den der Führungsdraht eines Kathetersystems geführt wird. Dem kanalbildenden Kopplungselement 4a liegt zumindest abschnittsweise eine Membran 4b zur Verstärkung des vibroakustischen Signals an.

Das Kopplungselement 4a ist entsprechend indirekt mit dem mindestens einen Sensor 4c verbunden und geeignet, vibroakustische Signale möglichst störungs- und verlustfrei von dem Instrument 2 an den mindestens einen Sensor 4c zu übertragen. Bei dieser indirekten Verbindung ist das Kopplungselements 4a nicht direkt mit einem Sensor 4c, sondern mit einer Membran 4b verbunden, die die vibroakustischen Signale in einen sich an die Membran 4b anschließenden Schallraum überträgt. Die Membran 4b ist geeignet, die vom Kopplungselement 4a übertragenen vibroakustischen Signale zu verstärken, wobei der Schallraum zu einer zusätzlichen Verstärkung des Signals führt. Grundsätzlich ist es auch denkbar, dass die vibroakustischen Signale direkt, also ohne eine zwischengeschaltete Membran 4b, vom Kopplungselement 4a in den Schallraum übertragen werden.

Bei einer indirekten Verbindung ist der mindestens eine Sensor 4c vorzugsweise ein Luftmikrofon.

Die vom Sensor 4c erfassten Signale werden optional zunächst auf analoger Ebene verarbeitet 4d und anschließend einem Analog-Digital-Wandler 4e zugeführt. Ein Controller 4f steuert die Vorgänge und eine Kommunikationseinheit 4g regelt die kabellose oder kabelgebundene Übertragung der digitalen Daten an die Daten-/Signalverarbeitungsvorrichtung 5. Von der Daten-/Signalverarbeitungsvorrichtung 5 werden die Ergebnisse an die Ausgabevorrichtung 6 übertragen.

Bei dieser Ausführungsform ist die Signalerfassungsvorrichtung 4 vorzugsweise getrennt von der Daten-/Signalverarbeitungsvorrichtung 5 und der Ausgabevorrichtung 6 untergebracht.

Figur 7 zeigt eine sechste Ausführungsform der erfindungsgemäßen Vorrichtung speziell zur Verwendung bei Roboter-assistierten Eingriffen (RAS) und dort insbesondere zur Anbringung an der Oberfläche eines laparoskopischen Instruments 2. Die erfindungsgemäße Vorrichtung umfasst in dieser Ausführungsform eine Signalerfassungsvorrichtung 4 mit einem Kopplungselement 4a zum Festlegen zumindest der Signalerfassungsvorrichtung 4 am proximalen Ende eines medizinischen Instruments 2, nämlich eines laparoskopischen Instruments bei der RAS. Das Kopplungselement 4a ist mit seinem proximalen Ende indirekt mit dem mindestens einen Sensor4c verbunden und geeignet, vibroakustische Signale möglichst störungs- und verlustfrei von dem Instrument 2 an den mindestens einen Sensor 4c zu übertragen. Bei dieser indirekten Verbindung ist das proximale Ende des Kopplungselements 4a nicht direkt mit einem Sensor 4c, sondern vorzugsweise mit einer Membran 4b verbunden, die die vibroakustischen Signale in einen sich an die Membran 4b anschließenden Schallraum überträgt. Die Membran 4b ist geeignet, die vom Kopplungselement 4a übertragenen vibroakustischen Signale zu verstärken, wobei der Schallraum zu einer zusätzlichen Verstärkung des Signals führt. Grundsätzlich ist es auch denkbar, dass die vibroakustischen Signale direkt, also ohne eine zwischengeschaltete Membran 4b, vom Kopplungselement 4a in den Schallraum übertragen werden.

Bei einer indirekten Verbindung ist der mindestens eine Sensor 4c vorzugsweise ein Luftmikrofon.

Die vom Sensor 4c erfassten Signale werden optional zunächst auf analoger Ebene verarbeitet 4d und anschließend einem Analog-Digital-Wandler 4e zugeführt. Ein Controller 4f steuert die Vorgänge und eine Kommunikationseinheit 4g regelt die kabellose oder kabelgebundene Übertragung der digitalen Daten an die Daten-/Signalverarbeitungsvorrichtung 5. Von der Daten-/Signalverarbeitungsvorrichtung 5 werden die Ergebnisse an die Ausgabevorrichtung 6 übertragen.

Bei dieser Ausführungsform ist die Signalerfassungsvorrichtung 4 vorzugsweise getrennt von der Daten-/Signalverarbeitungsvorrichtung 5 und der Ausgabevorrichtung 6 untergebracht.

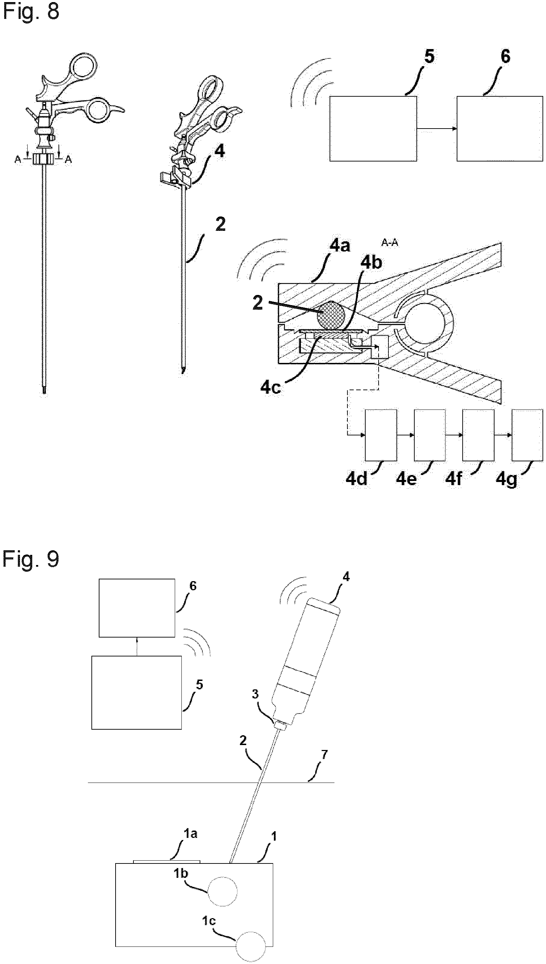

Figur 8 zeigt eine siebte Ausführungsform der erfindungsgemäßen Vorrichtung speziell zur Verwendung mit einem stabförmigen laparoskopischen Instrument. Die erfindungsgemäße Vorrichtung umfasst in dieser Ausführungsform eine Signalerfassungsvorrichtung 4 mit einem Kopplungselement 4a zum Festlegen zumindest der Signalerfassungsvorrichtung 4 vorzugsweise am proximalen Ende eines medizinischen Instruments 2, nämlich eines stabförmigen laparoskopischen Instruments. Das Kopplungselement 4a ist zangenartig ausgebildet und kann in der Art einer Wäscheklammer mit einem Ende an dem stabförmigen Abschnitt 2 des laparoskopischen Instruments festgelegt werden. In diesem klammerartigen Abschnitt des Kopplungselements 4a ist zumindest abschnittsweise eine Membran 4b vorgesehen, die in Kontakt mit dem stabförmigen Abschnitt 2 des laparoskopischen Instruments ist. Die Membran 4b ist geeignet, vibroakustische Signale möglichst störungs- und verlustfrei von dem Instrument 2 an den mindestens einen Sensor 4c zu übertragen. Bei dieser indirekten Verbindung ist das proximale Ende des Kopplungselements 4a nicht direkt mit einem Sensor 4c, sondern vorzugsweise mit einer Membran 4b verbunden, die die vibroakustischen Signale verstärkt an einen Sensor 4c weiterleitet. Die Membran 4b ist geeignet, die vom Instrument 2 beziehungsweise gegebenenfalls auch vom Kopplungselement 4a übertragenen vibroakustischen Signale zu verstärken.

Bei dieser indirekten Verbindung ist der mindestens eine Sensor 4c vorzugsweise ein Piezo- oder Kristallmikrofon.

Die vom Sensor 4c erfassten Signale werden optional zunächst auf analoger Ebene verarbeitet 4d und anschließend einem Analog-Digital-Wandler 4e zugeführt. Ein Controller 4f steuert die Vorgänge und eine Kommunikationseinheit 4g regelt die kabellose oder kabelgebundene Übertragung der digitalen Daten an die Daten-/Signalverarbeitungsvorrichtung 5. Von der Daten-/Signalverarbeitungsvorrichtung 5 werden die Ergebnisse an die Ausgabevorrichtung 6 übertragen.

Bei dieser Ausführungsform ist die Signalerfassungsvorrichtung 4 vorzugsweise getrennt von der Daten-/Signalverarbeitungsvorrichtung 5 und der Ausgabevorrichtung 6 untergebracht.

Figur 9 zeigt die Palpation, bei der das Abtasten einer Gewebestruktur 1 mit der an eine Abtastsonde 2 über ein Adapterelement 3 angeschlossenen Signalerfassungsvorrichtung 4 der erfindungsgemäßen Vorrichtung über die Datenverarbeitungsvorrichtung 5 und die Ausgabevorrichtung 6 audifiziert wird.

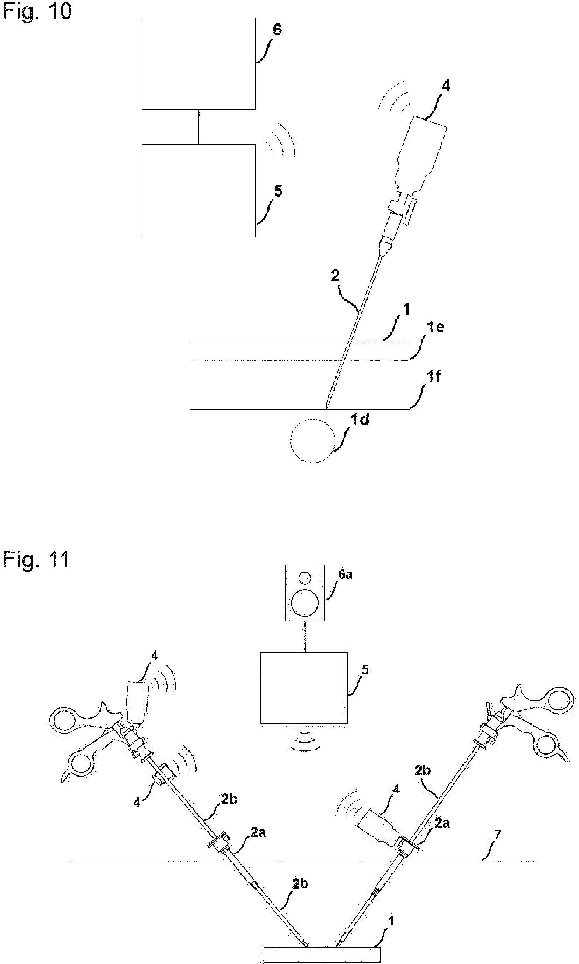

Figur 10 zeigt eine perkutane Nadeleinführung, bei der die Signalerfassungsvorrichtung 4 der erfindungsgemäßen Vorrichtung an eine Insufflationsnadel 2 vom Typ Veres angeschlossen ist und das Durchdringen einzelner Gewebeschichten 1e, 1f, 1d über die Datenverarbeitungsvorrichtung 5 und die Ausgabevorrichtung 6 audifiziert wird.

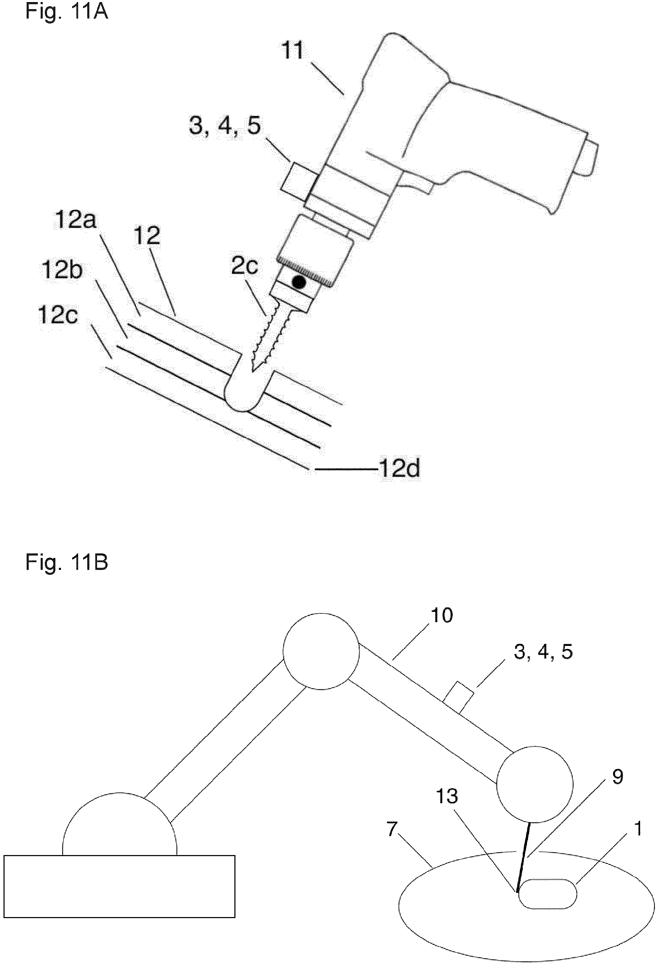

Figur 11 zeigt einen laparoskopischen Eingriff mit mehreren Signalerfassungsvorrichtung 4, die jeweils mit den verwendeten Instrumenten 2 verbunden sind, um die Gewebe-Instrument-Interaktionen 1 während des Eingriffs innerhalb des Körpers 7 zu überwachen.

Figur 11A zeigt eine erfindungsgemäße Vorrichtung für eine spezifische Anwendung zur vibroakustischen Echtzeit-Überwachung für präzise Knochenbohrungen und Verschraubungen bei chirurgischen Eingriffen.

Die Signalerfassungsvorrichtung 4 ist über ein optionales Adapterelement 3 an dem medizinischen Instrument, hier an einem Teil einer chirurgischen Bohrmaschine 11, beispielsweise auch dem Bohrfutter, mit der Daten-/ Signalverarbeitungsvorrichtung 5 verbunden. Die chirurgische Bohrmaschine 11 umfasst einen Bohrer 2c, mit dem in Knochenmaterial 12 gebohrt werden kann. Die verschiedenen Knochenschichten mit unterschiedlichen Knochendichten 12a bis 12d können anhand der vibroakustischen Signale, die diese impliziert durch den Bohrer aussenden, und das Durchbrechen der abschließenden Knochenschicht detektiert werden.

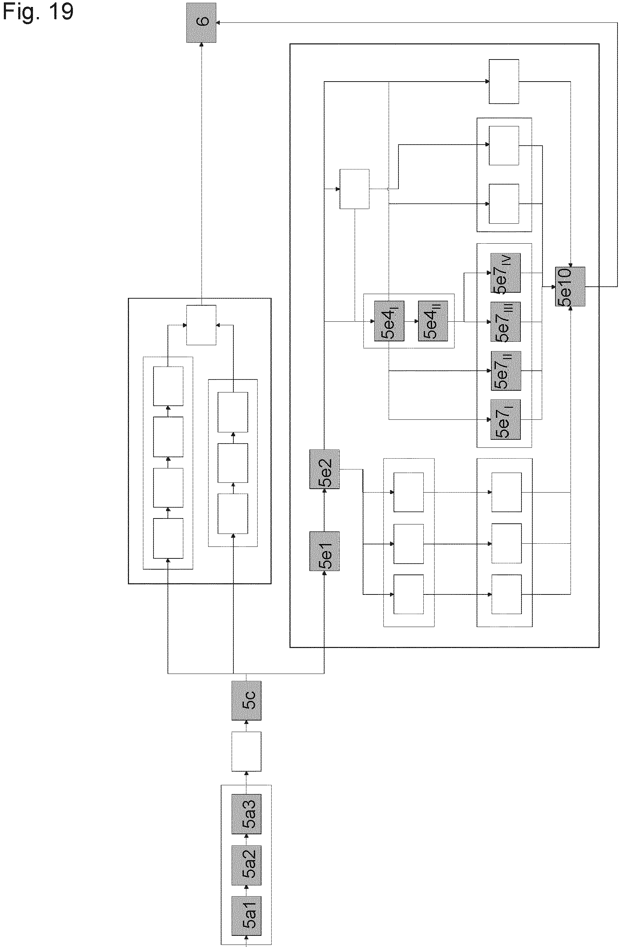

Weitere Einzelheiten finden sich in der Beschreibung zu Figur 19.

Figur 11B zeigt das Flussdiagramm für die Datenverarbeitung für eine spezifische Anwendung zur vibroakustischen Echtzeit-Überwachung zur Erkennung von Kollisionen bei chirurgischen Eingriffen.

Die Signalerfassungsvorrichtung 4 ist über ein optionales Adapterelement 3 an dem medizinischen Instrument, hier einem chirurgischen Roboter beziehungsweise Roboterarm 10, mit der Daten-/ Signalverarbeitungsvorrichtung 5 verbunden. Das chirurgische Instrument 9, das an dem Roboterarm 10 festgelegt ist, soll möglichst nicht mit dem Körper 7 beziehungsweise dem Gewebe 1 kollidieren. Eine auch nur kleinste Kollision 13 kann anhand vibroakustischer Signale erfasst und dem Anwender oder dem Roboter (-arm) als Steuersignal gemeldet werden.

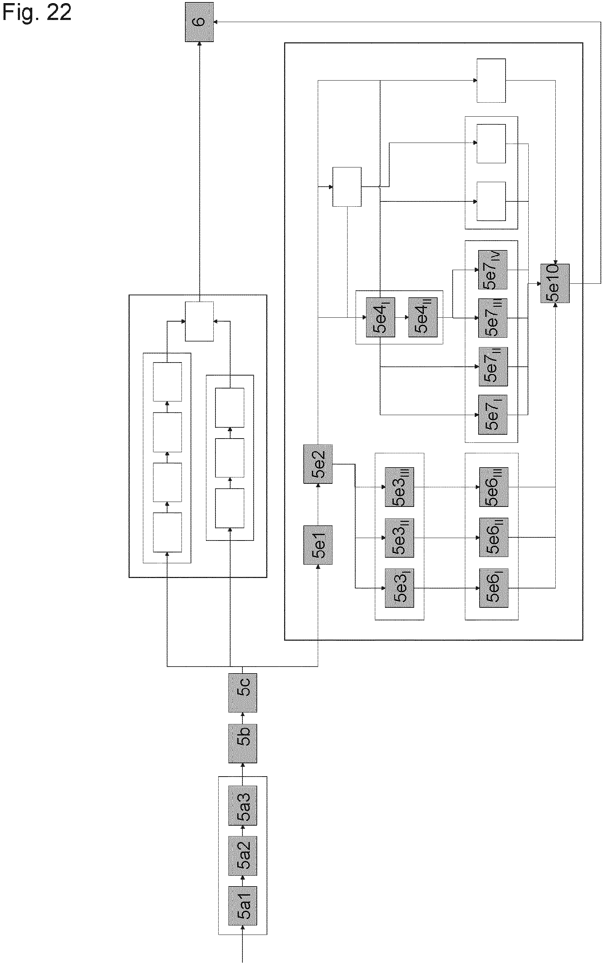

Weitere Einzelheiten finden sich in der Beschreibung zu Figur 22.

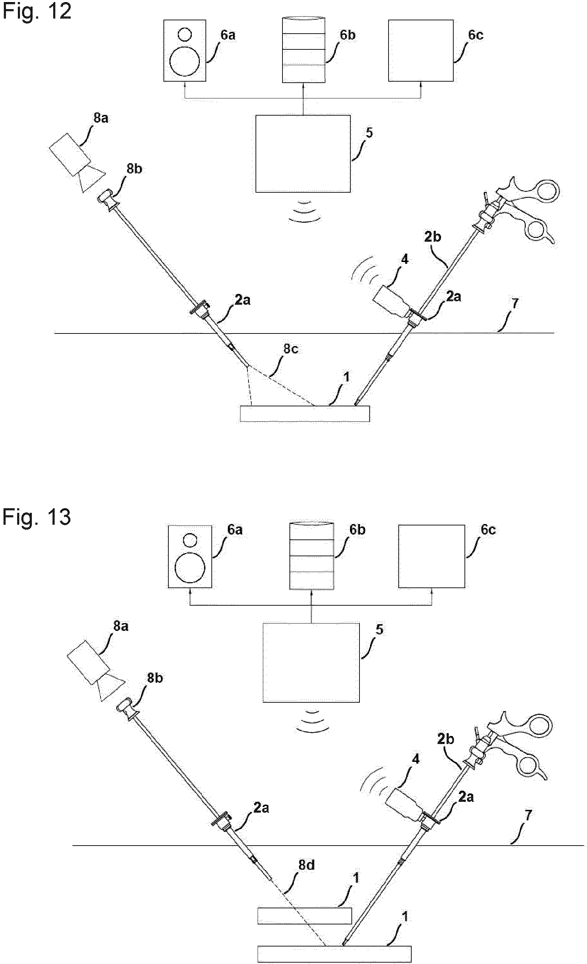

Figur 12 zeigt einen laparoskopischen Eingriff, bei dem die Signalerfassungsvorrichtung 4 mit dem laparoskopischen Port (Trokar) 2a verbunden ist und indirekt die Interaktion des eingeführten laparoskopischen Instruments 2b erfasst, um Interaktionen außerhalb des Sichtfelds 8c (FOV) des endoskopischen Kamerasystems 8a zu erkennen und zu nutzen.

Figur 13 zeigt einen laparoskopischen Eingriff, bei dem die Signalerfassungsvorrichtung 4 mit dem laparoskopischen Port (Trokar) 2a verbunden ist und indirekt die Interaktion des eingeführten laparoskopischen Instruments 2b erfasst, um Interaktionen außerhalb der Sichtlinie 8d (LOS) des endoskopischen Kamerasystems 8a zu erkennen und zu nutzen.

Figur 14 zeigt das grundsätzliche Flussdiagramm des erfindungsgemäßen Verfahrens zur Datenverarbeitung vibroakustischer Signale. Nicht für alle Anwendungen werden alle Verfahrensschritte genutzt oder sind diese notwendig. Die folgenden Figuren enthalten spezielle Anwendungen des Verfahrens. Nachfolgend sollen entsprechend vor allem die einzelnen möglichen Schritte kurz erläutert werden. Der genaue Verarbeitungsfluss ergibt sich aus der Figur.

Das erfindungsgemäße computerimplementierte Verfahren umfasst in einem ersten, nicht dargestellten Schritt, grundsätzlich zunächst das Bereitstellen vorhandener digitalisierter vibroakustischer Signale.

Der grundsätzliche Verfahrensablauf des erfindungsgemäßen computerimplementierten Verfahrens umfasst entsprechend die folgenden Schritte:
(0) Bereitstellen eines vorhandenen digitalisierten vibroakustischen Signals;
(I) Signalvorverarbeitung des vibroakustischen Signals (5a); und
(II) Identifizierung von Segmenten/Regionen von Interesse (5b); und
(III) Audifikation (5d); und/oder (5c)
(IV) Klassifizierung der Segmente/Regionen von Interesse (5e).

Optional erfolgt in einem weiteren Schritt (V) beispielsweise die Ausgabe, Weiterverarbeitung, Speicherung etc. (6) der klassifizierten Segmente/Regionen.

Die Vorverarbeitung *(Pre-processing)* 5a zielt darauf ab, die verschiedenen Störgeräusche im VA-Signal abzuschwächen und die Interaktionsereignisse zwischen Instrumentenspitze und Schaft sowie Gewebe zu verstärken. Zunächst werden die kontinuierlichen Komponenten (DC) aus dem VA-Signal entfernt 5a1. Dann wird ein Schritt zur Reduzierung des Hintergrundrauschens 5a2 durchgeführt. Dies kann zum Beispiel durch spektrale Gating-Methoden erfolgen. Schließlich wird ein Schritt zur Signalverbesserung 5a3 durchgeführt, um die Dynamik und die Frequenzkomponenten im Zusammenhang mit den Interaktionen zu verbessern. Dies kann mit linearen Filtern, adaptiven Filtern oder mit Wavelet-basierten Filtern geschehen. Bei Wavelet-basierten Filtern kann die diskrete Wavelet-Transformation (DWT) verwendet werden, um das Signal in verschiedene Skalen zu zerlegen und dann das Signal anhand der Skalen zu rekonstruieren, in denen interessierende Ereignisse auftreten, dies können beispielsweise Wechselwirkungen mit hoher Energie sein.

Die Identifizierung von Segmenten/Regionen von Interesse (*Identification of segments*/*regions of interest*) 5b kann verwendet werden, um interessante Segmente und Regionen im Signal zu segmentieren, in denen wichtige Interaktionsereignisse zwischen Instrument und Gewebe/Oberfläche auftreten. Dies kann dazu dienen, eine Audifikation oder Klassifizierung durchzuführen, die sich nur auf die relevanten Interaktionsinformationen konzentriert. Zu diesem Zweck können der Beginn und die Verschiebung eines möglichen Interaktionsereignisses beispielsweise mit Algorithmen zur Erkennung abrupter Veränderungen (zum Beispiel mit dem Hypothesentest des Hinkley-Page-Tests) oder mit Algorithmen zur Erkennung von Ableitungen ermittelt werden.

### Nicht-ausschließlicher ODER-Operator (Non-exclusive OR operator) 5c

Dieser Operator deutet auf die Möglichkeit hin, den Weg der Audifikation und/oder den Weg der Klassifizierung zu gehen. Für einige Anwendungen kann nur eine Audifikation als Feedback zur Verfügung gestellt werden und für einige andere Anwendungen ist nur eine Klassifizierung notwendig. Es gibt auch Anwendungen, die eine Integration von Klassifizierung und Audifikation von Interaktionsereignissen benötigen.

### Audifikation (Audification) 5d

- Direkte Audifikation (*Direct audification*) 5d1: Hierbei handelt es sich um eine direkte Umwandlung des VA-Signals in einen hörbaren Klangbereich unter Verwendung verschiedener Standardverarbeitungswerkzeuge wie Filterung, Verstärkungsoperatoren oder Effektgenerierung. Sie kann vier Hauptteilschritte umfassen:
   o Filterung des Signals (*Filtering*) 5d1_{I}: Das Hauptziel dieses Schritts ist die Verbesserung der Frequenzkomponenten von Signalereignissen oder Signalsegmenten von Interesse, die mit den für ein bestimmtes Verfahren signifikanten Wechselwirkungen zwischen Spitze/Schaft und Gewebe/Oberfläche zusammenhängen. Diese Filterstufe kann mit verschiedenen Techniken durchgeführt werden, z. B. mit der linearen Filterung, der adaptiven Filterung oder der Filterung, die mit einer beliebigen Art von Signalzerlegungstechniken erzeugt wird, wie z. B. der kontinuierlichen Wavelet-Transformation (CWT), der diskreten WaveletTransformation (DWT), der empirischen Modedekomposition (EMD) oder der autoregressiven Modellierung. Das Signal wird in verschiedene Skalen, Modi oder Dynamiken zerlegt (je nach verwendeter Technik) und dann unter Verwendung von Skalen/Modi/Dynamiken rekonstruiert, in denen Interaktionsereignisse mit hoher Energie vorhanden sind.
   ∘ Lautstärkeanpassung (*Volume adjustment*) 5d1_{II}: Diese besteht aus der Anwendung einer Verstärkung auf das Signal, um die Lautstärke zu erhöhen. Sie kann auch zur Begrenzung zu lauter Ereignisse oder gesättigter Audiosignalsegmente verwendet werden.
   ∘ Dynamikbearbeitung (*Dynamic processing*) 5d1_{III}: Mit diesem Schritt werden die dynamischen Eigenschaften des Audiosignals verändert. Er ermöglicht die Einstellung eines Verhältnisses zwischen den Eingangspegeln und den Ausgangspegeln. Dies kann in einem einzigen Frequenzband oder in mehreren Frequenzbändern für eine unabhängige, parallele Dynamikbearbeitung erfolgen.
   ∘ Erzeugung von Effekten (*Effects generation*) 5d1 iv: Ziel ist es, den Klang des Audiosignals zu manipulieren. Übliche Effekte, die hier verwendet werden können, sind Nachhall, Hall, Faltung, Echo, Chorus, Zeitdehnung, Transposition, Harmonizer.
- Indirekte Audifikation (*Indirect Audification*) 5d2: Dieser Schritt umfasst die Verknüpfung von Merkmalen des VA-Signals mit auditiven Parametern zum Zweck der Datendarstellung. Er besteht aus der Extraktion von Merkmalen, die dann in eine auditive Darstellung umgewandelt werden, indem die Merkmale verschiedenen Parametern des angezeigten Tons zugeordnet werden. Er umfasst drei Hauptunterschritte:
   ∘ Merkmalsextraktion (*Feature extraction*) 5d2_{I}: In der ersten Phase werden verschiedene Merkmale oder Attribute aus dem Signal extrahiert. Es können verschiedene Arten von Merkmalen aus dem Signalsegment extrahiert werden, wie z. B. Merkmale im Zeitbereich, im Frequenzbereich oder im Zeit-Frequenz-Bereich. Beispiele für Merkmale im Zeitbereich sind Signalstärken oder -energien, statistische Merkmale wie Signalmittelwert oder -varianz und morphologische Merkmale. Für Merkmale im Frequenzbereich können Schätzungen des Signalspektrums mit verschiedenen Spektralschätzungsmethoden wie FFT, AR-Modellierung usw. durchgeführt werden. Für Merkmale im Zeit-/Frequenzbereich können Methoden wie die Kurzzeit-FourierTransformation (STFT), das Mel-Frequenz-Cepstrum, die Wigner-Ville-Verteilung, die kontinuierliche Wavelet-Transformation (CWT), die diskrete Wavelet-Transformation (DWT), die empirische Modenzerlegung (EMD), die zeitvariable autoregressive Modellierung, Zyklostationaritätsmerkmale usw. verwendet werden.
   ∘ Mapping (*Mapping*) 5d2_{II}: Die Merkmale werden dann in Audioparameter umgewandelt, die zur Erzeugung einer auditiven Darstellung dienen. Dies kann geschehen
      ▪ durch eine direkte Zuordnung der extrahierten Merkmale über eine Parameter-Mapping-Sonifikation, bei der z. B. Änderungen der Signalstärke auf Änderungen der Tonhöhe der resultierenden akustischen Darstellung abgebildet werden können;
      ▪ durch ein dynamisches Modell, das die zeitliche Entwicklung eines Systems beschreibt, das durch die Merkmale angeregt wird, wobei das Modell eine physikalische Schallquelle simulieren soll;
      ▪ mit Hilfe von Entscheidungsregeln, Clustermethoden oder einem trainierten maschinellen Lernmodell, das die Merkmale als Eingaben verwendet und klassifizierte Ereignisse als Ausgaben erzeugt, die dann für die Parametrisierung des Geräuschs verwendet werden.
   ∘ Klangsynthese (*Sound synthesis*) 5d2_{III}: Erzeugung von Klangeigenschaften.
- Tonprojektion (*Sound projection*) 5d3: Erzeugt alles Notwendige für die Übertragung und/oder Ausgabe der erzeugten Tonausgabe an der Ausgabevorrichtung (6), über einen oder mehrere Lautsprecher oder Kopfhörer.

Klassifizierung der Segmente/Regionen von Interesse *(Classification)* 5e
- Identifizierung transienter und stationärer Dynamik (*Transient and stationary dynamics identification*) 5e1: Die aus den Wechselwirkungen zwischen Instrument und Oberfläche resultierenden VA-Signale weisen eine sehr komplexe Dynamik auf. Ein einziges Interaktionsereignis kann in einem sehr kurzen Zeitintervall eine Mischung aus transienter und stationärer Dynamik aufweisen. Zum Beispiel kann bei einem Tastvorgang in dem Moment, in dem die Wenn die Spitze des Instruments in Kontakt mit der VA-Anregung kommt, führt dies zu einer extrem kurzzeitigen transienten Reaktion, gefolgt von einer stationären Reaktion, die sich aus dem effektiven Abtastvorgang ergibt. Inhomogenitäten der Oberfläche können zu Kollisionen der Spitze mit der Oberfläche führen, was wiederum zu einer Mischung aus transienter und stationärer Dynamik führt. In dem Moment, in dem die Palpation aufhört, kann auch der Kontaktstopp eine transiente Dynamik erzeugen, so dass in einem kurzen Zeitintervall eine Reihe von transienten-stationären Dynamiken auftreten können. Diese Komplexität führt dazu, dass das Signal nicht mit Standard- oder klassischen Signalverarbeitungsalgorithmen verarbeitet werden kann, die normalerweise für Sprache oder Musiktöne verwendet werden, deren akustische Antworten in einem kurz- bis mittelfristigen Zeitintervall recht stabil sein können.
- Spektralbandzerlegung (*Spectral band decomposition*) 5e2: In diesem Schritt wird das VA-Signal in mehrere Schmalbandsignale zerlegt, indem das Gesamtfrequenzband des Signals in mehrere Teilfrequenzbänder zerlegt wird. Das Hauptziel besteht darin, später verschiedene Aspekte des Signals getrennt zu verarbeiten. Wir gehen davon aus, dass sich eine Bildtextur aus mehreren Dynamiken zusammensetzt, die Unregelmäßigkeitseigenschaften der Textur wie Glätte oder Rauheit repräsentieren. Daher können die Signale in mehrere Dynamiken zerlegt werden, die z. B. die Stufen der Unregelmäßigkeiten in der Interaktion darstellen können. Die Anzahl der Frequenzbänder, in die das Signal zerlegt wird, hängt davon ab, ob das Signal durch Einführen, Abtasten oder eine andere Art der Interaktion zwischen Instrument und Gewebe/Oberfläche entsteht. Die Anzahl der Bänder kann zwischen 2 und 10 Frequenzteilbändern variieren. Die Zerlegung kann mit verschiedenen Methoden durchgeführt werden, z. B. Filterbanken, kontinuierliche Wavelet-Transformation (CWT), diskrete Wavelet-Transformation (DWT), Polverfolgung und zeitvariante autoregressive Modellierung, empirische Modedekomposition (EMD) usw.
- Berechnung von Indikatoren (*Indicators computation*): Vor dem Schritt der Merkmalsextraktion ist es notwendig, zunächst Indikatoren aus den Signalen zu extrahieren. Diese Indikatorberechnung erhält als Eingabe normalerweise ein oder mehrere Signale und liefert als Ausgabe ein oder mehrere Signale oder Matrizen. Im Rahmen der erfindungsgemäßen Signalverarbeitungsmethodik werden drei Arten von Indikatoren vorgeschlagen:
   ∘ Zeitbereichsindikatoren (*Time domain indicators*) 5e3: Dies sind Indikatoren, die direkt aus den Zeitreihen berechnet werden. Es können drei Arten von Zeitbereichsindikatoren berechnet werden:
      ▪ Statistische Indikatoren (*Statistical indicators*) 5e3_{I}: Indikatoren, die durch statistische Standardoperationen berechnet werden, z. B. durch Schätzung der Wahrscheinlichkeitsverteilung (datenbasiert oder modellbasiert) oder durch Berechnung des zeitabhängigen Durchschnitts oder der Varianz, z. B. unter Verwendung eines zeitlich gleitenden Fensters.
      ▪ Morphologische Indikatoren (*Morphological indicators*) 5e3_{II}: Indikatoren, die verschiedene Aspekte der Morphologie des Signals betonen. Beispiele für diese Art von Indikatoren sind Ableitungen, Krümmungsindikatoren oder Ableitungsindikatoren zweiten Grades, Polynomanpassung, Schätzung des Überschwingens. Auch die Darstellung der ADSR-Hüllkurve kann als morphologischer Indikator verwendet werden.
      ▪ Auf plötzlichen Änderungen basierende Indikatoren (*Abrupt change-based indicators*) 5e3_{III}: Indikatoren, die Änderungen im Signal bewerten, die in einem kurzen Zeitintervall auftreten. Zum Beispiel kann in dem Moment, in dem das Instrument mit einer Oberfläche in Kontakt kommt, die abrupte Änderung des Signals Informationen über die Härte der Oberfläche liefern. Ein anderes Beispiel ist der Einstich einer Nadel in eine Schicht, bei dem die abrupte Änderung, die sich aus dem Bruch des Gewebes ergibt, mit der Elastizität des Gewebes und seiner Widerstandseigenschaft gegenüber der Instrumentenspitze in Verbindung gebracht werden kann. Die abrupte Veränderung kann beispielsweise mit Hilfe eines statistischen Hypothesentests wie dem Hinkley- und Page-Test oder dem CUSUM-Test bewertet oder berechnet werden.
   ∘ Indikatoren im Frequenz- und Zeit-Frequenz-Bereich (*Frequency and time-frequency domain indicators*) 5e4:
      ▪ Spektrum basierte Indikatoren (*Spectrum-based indicators*) 5e4_{I}: Indikatoren, die dem stationären Spektrum (beispielsweise ein Periodogramm) oder dem Zeit-Frequenz-Spektrum entsprechen (beispielsweise unter Verwendung von Spektrogramm, Wygner-Ville, CWT, zeitvarianter autoregressiver Modellierung, usw.).
      ▪ Indikatoren auf der Grundlage der momentanen dominanten Frequenz (*Instantaneous dominant frequency-based indicators*) 5e4_{II}: Indikatoren, die aus dem Zeit-Frequenz-Spektrum als augenblickliche dominante Frequenz berechnet werden, d. h. die Frequenz, die zu jedem Zeitpunkt die maximale Energie im Zeit-Frequenz-Spektrum enthält. Diese kann direkt aus der Zeit-Frequenz-Matrix oder parametrisch über ein Zeitvariantenmodell wie bei TVAR unter Verwendung von Polschätzung/- verfolgung berechnet werden.
   ∘ Hüllkurvenbasierte Indikatoren (*Envelope-based indicators*) 5e5: Extrahierte Indikatoren, die der Hüllkurve des Signals entsprechen. Die Hüllkurve kann beispielsweise mittels DWT oder Hilbert-Transformation mit oder ohne homomorphe Filterung berechnet werden.
- Merkmalsberechnung (*Features computation*): Es können vier Merkmalsfamilien extrahiert werden, die in insgesamt zehn Unterfamilien unterteilbar sind. Jedes Merkmal entspricht einer skalaren Zahl.
   ∘ Zeitbereichsmerkmale (*Time-domain features*) 5e6: Sie erhalten als Eingabe einen Zeitbereichsindikator und berechnen verschiedene Familien von Merkmalsskalarwerten.
      ▪ Statistische Merkmale im Zeitbereich (*Time-domain statistical features*) 5e6_{I}: Berechnung klassischer statistischer Werte aus einem Indikator, z. B. Durchschnittswerte, Varianz, Kurtosis, Schiefe usw.
      ▪ Morphologische Merkmale im Zeitbereich (*Time-domain morphological features*) 5e6_{II}: Berechnung morphologischer Werte aus den Indikatoren wie Steigungen, maximale Steigungen, Krümmungen, Latenzen, Dauer der Segmente und Überschwinger. Das Hauptziel dieser Familie von Merkmalen ist die Bewertung der Morphologie der VA-Wellenerregung im Zeitverlauf, die aus einem Interaktionsereignis resultiert.
      ▪ Auf abrupten Veränderungen basierende Merkmale im Zeitbereich (*Time-domain abrupt change based features*) 5e6_{III}: Diese Familie von Merkmalen zielt darauf ab, die Größe der Veränderung zu Beginn oder am Ende eines Interaktionsereignisses oder einige abrupte Veränderungen zu quantifizieren, die während eines Interaktionsereignisses aufgrund von Inhomogenitäten im Gewebe/an der Oberfläche auftreten. Höhere Werte dieser Indikatoren könnten auf härteres Gewebe hindeuten und andersherum.
   o Spektralbereich-Merkmale (*Spectral domain features*) 5e7: Berechnung von Merkmals-Skalarwerten aus dem stationären Spektrum (Vektor) oder aus dem Zeit-Frequenz-Spektrum (Matrix).
      ▪ Zeit-Frequenz-Spektrum-Ereignis-Merkmale (*Time frequency spectrum events features)* 5e7_{I}: Werte im Zusammenhang mit Ereignissen, die im Zeit-Frequenz-Spektrum identifiziert werden können. Beispiele für Ereignisse sind maximale Spektralenergien, abrupte Änderungen der Spektralenergien, abrupter Energieübergang von einer Frequenz zur anderen, Wechsel von nicht stationären zu stationären Frequenzkomponenten usw.
      ▪ Spektralenergiemerkmale (*Spectral energy features*) 5e7_{II}: Merkmale, die sich auf die Energien im Spektrum beziehen. Sie können als absolute oder relative Energien berechnet werden. Die relativen Energien können Verhältnisse zwischen den Energien in verschiedenen Bändern sein (z. B. berechnet aus 5e2) oder relativ zur Gesamtenergie des Signals sein. Diese Familie von Merkmalen gibt Aufschluss über die spektrale Verteilung der an der Wechselwirkung beteiligten spektralen Energien. Wenn beispielsweise eine Nadel eine Gewebeschicht durchbricht, werden im Allgemeinen hochfrequente Komponenten angeregt. Daher werden in diesem Fall die Energieverhältnisse im Hochfrequenzbereich hervorgehoben. Bei der Palpation sind hohe Frequenzen beispielsweise charakteristisch für Wechselwirkungen mit hartem Gewebe und niedrige Frequenzen für Wechselwirkungen des Werkzeugs mit weichem Gewebe.
      ▪ Statistische Merkmale der momentanen dominanten Frequenz (*Instantaneous dominant frequency statistical features*) 5e7_{III}: Berechnung klassischer statistischer Indikatoren aus dem Indikator der momentanen dominanten Frequenz, wie Mittelwerte und Varianz. Statistische Werte der momentanen dominanten Frequenz können direkt mit der Homogenität der interagierenden Oberfläche oder des Gewebes in Verbindung gebracht werden. Eine hohe Varianz der momentanen dominanten Frequenz zeigt beispielsweise an, dass das interagierende Gewebe sehr inhomogen ist, und eine geringe Varianz kann ein Merkmal eines glatten Gewebes oder eines Gewebes sein, dessen Eigenschaften sich im Raum nicht wesentlich ändern.
      ▪ Dynamische Merkmale der momentanen dominanten Frequenz (*Instantaneous dominant frequency dynamical features*) 5e7_{IV}: Merkmale, die darauf abzielen, das zeitliche Verhalten der momentanen dominanten Frequenz zu beschreiben. Eine dominante Frequenz kann in der Zeitdomäne oder in ihrem zeitlichen Verlauf der komplexen Zahlendarstellung 2d verfolgt werden. Im Zeitbereich wirkt die dominante Frequenz wie ein Signal, dessen Dynamik (oszillierend, stationär, transient usw.) als Werte extrahiert werden kann. In der 2d komplexen Darstellung kann eine dominante Frequenz als Teilchen fungieren, das sich in einem 2d-Raum bewegt. Dieses Teilchen beinhaltet eine Bewegung in der Zeit, die quantifiziert und in Merkmalswerte umgewandelt werden kann.
   o Hüllkurvenbasierte Merkmale (*Envelope-based features*) 5e8: Merkmale, die direkt aus der Hüllkurve im Zeitbereich oder aus einer spektralen Transformation der Hüllkurve berechnet werden. Manchmal handelt es sich um zwei komplexe Interaktionen, die ein hohes Maß an nichtlinearer Dynamik aufweisen und stark moduliert sind (beispielsweise Bohren oder Abtasten stark inhomogener Strukturen), und sie können mit Hilfe der Einhüllenden des Signals vereinfacht werden.
      ▪ Spektrale Hüllkurvenmerkmale (*Spectral envelope features*) 5e8_{I}: Merkmale, die aus dem stationären oder zeitlichen Frequenzspektrum der Hüllkurve berechnet werden. Diese Merkmale werden verwendet, um nichtlineare Merkmale der Interaktion zu extrahieren.
      ▪ Hüllkurvenbasierte Merkmale im Zeitbereich (*Time-domain envelope-based features*) 5e8_{II}: Merkmale, die direkt aus dem Hüllkurvenindikator berechnet werden. Sie können wichtige Informationen über Dämpfung und Unregelmäßigkeiten im interagierenden Gewebe liefern. So kann beispielsweise die Anzahl der Spitzen und Täler der Hüllkurve Aufschluss über Unebenheiten geben, die durch die Interaktion zwischen Werkzeug und Gewebe/Oberfläche entstehen. Diese Unebenheiten können mit starken Unregelmäßigkeiten zusammenhängen, die dem Gewebe innewohnen können oder, im Falle der Palpation, mit darunter liegenden Strukturen zusammenhängen können.
   o Deep-Learning-basierte Merkmale (*Deep learning based features*) 5e9: Deep-Learning-basierte Merkmale werden von einem mehrschichtigen neuronalen Netz berechnet, das die Daten auf nichtlineare Weise verarbeitet. Zu diesem Zweck können die Spektralbandzerlegung (5e2) oder die spektrumbasierten Indikatoren (5e4_{I}) als Eingangsdaten in den vorgesehenen Deep-Learning-Algorithmus eingespeist werden, z. B. in mehrschichtige Perzeptronen (MLPs), Faltungsneuronale Netze (CNN), rekurrente neuronale Netze (RNNs), Langzeitspeichernetze (LSTMs), Autoencoder und eingeschränkte Boltzmann-Maschinen (RBM). Jeder dieser Algorithmen kann auf der Grundlage der Charakterisierung der Eingabedaten automatisch eine Reihe von Merkmalen auf höherer Ebene erlernen.
- Trainiertes Modell des maschinellen Lernens (*Machine learning trained model*) 5e10: Die extrahierten Merkmale können in einen Vektor eingeordnet werden, der in ein trainiertes Modell des maschinellen Lernens eingespeist wird, das mit verschiedenen Architekturen wie Support Vector Machine (SVM), k-Nearest Neighbors (kNN), Random Forest, neuronalen Netzen und vielen anderen Ansätzen erstellt werden kann. Das trainierte Machine-Learning-Modell ordnet die Interaktionsereignisse des Eingangssignals VA vordefinierten Klassen zu. Beispiele für Klassen sind:
   o Bei Palpationsereignissen: verschiedene Klassen von Weichheit/Härte oder Rauheit/Glätte des Gewebes.
   o Bei Insertionsereignissen: verschiedene Klassen von Insertionsgewebe (Fett, Muskel, Bindegewebe usw.) oder verschiedene Klassen von Schichten wie Faszien, Peritoneum usw.
   o Bei laparoskopischen Eingriffen könnten die Klassen beispielsweise für verschiedene Aufgaben (Greifen, Schneiden, Abtasten, Nähen usw.) angegeben werden, oder die Klassen könnten die verschiedenen Instrumente sein, die erkannt werden können, wenn sie mit dem Gewebe/der Oberfläche in Berührung kommen (Greifer, Pinzette, Schere usw.)
- Schließlich könnte die Identifizierung der verschiedenen Klassen in der Ausgabevorrichtung 6 auch für verschiedene Zwecke wie Überwachung, Unterstützung, Speicherung usw. gespeichert oder angezeigt werden.

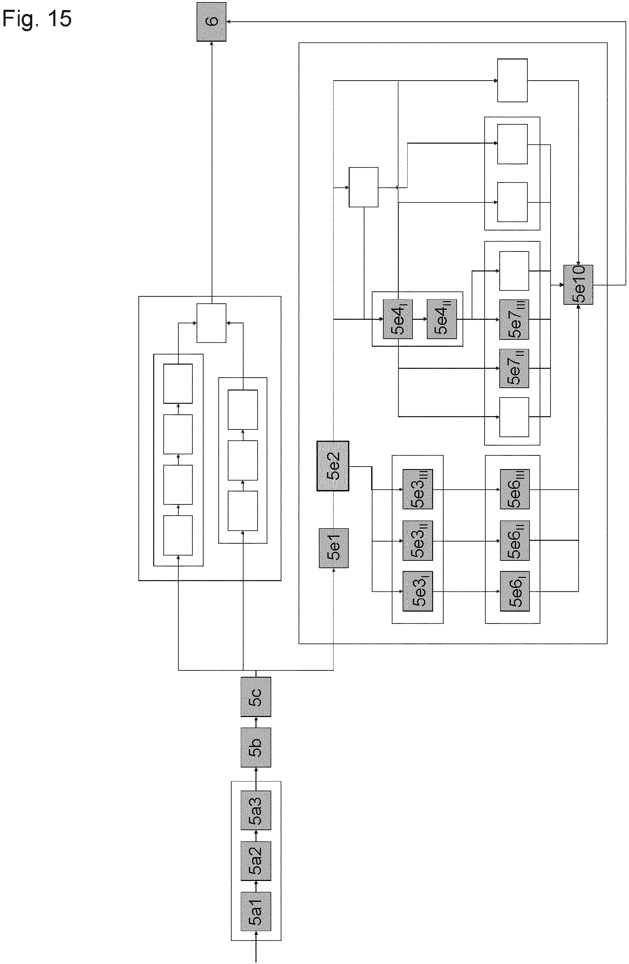

Figur 15 zeigt grau unterlegt die Blöcke, die für das erfindungsgemäße Verfahren im Zusammenhang mit einer Palpation aus dem allgemeinen Verarbeitungsdiagramm von Figur 14 verwendet werden.

Das allgemein beschriebene Verfahren kann für spezifische Anwendungen durch die Anpassung einzelner Verfahrensschritte optimiert werden, beispielsweise zur Klassifizierung von Ereignissen wie der Gewebehärte/-weichheit bei der Palpation. Der grundlegende Erfindungsgedanke besteht darin, Signalmerkmale mit Gewebe beziehungsweise Oberflächen Weichheits- beziehungsweise Härteklassen zu verknüpfen. So ist es beispielsweise möglich, die Gewebeweichheit/-härte in verschiedene Klassen zu unterteilen, zum Beispiel von Klasse 1 für sehr weiches Gewebe bis Klasse 10 für sehr hartes Gewebe. Ein Algorithmus für maschinelles Lernen, der mit Geweben trainiert wurde, deren Weichheits-/Härtegrad bekannt ist, kann den definierten Klassen Signalmerkmale zuordnen, was zu einem durch maschinelles Lernen trainierten Modell führt, das in der Lage ist, ein Palpationsereignis automatisch in die verschiedenen Weichheits-/Härteklassen einzuordnen.

Das erfindungsgemäße Verfahren zur Verarbeitung von vibroakustischen Signalen, die bei einer Palpation entstehen, dient insbesondere der Merkmalsextraktion und Klassifizierung. Das Verfahren stellt dabei insbesondere einen Übersetzungsalgorithmus zur Verfügung, mit dessen Hilfe die vibroakustischen Signale in für die Aufgabe relevante Informationen übersetzt werden können. Solche Informationen können beispielsweise die Darstellung von Eigenschaften der Zielstruktur oder Führungsinformationen für medizinische Instrumente sein.

Ein bevorzugtes Verfahren zur Signal- beziehungsweise Datenverarbeitung, beispielsweise zur Klassifizierung der Gewebehärte bei der Palpation, umfasst die folgenden Schritte:
(0) Bereitstellen eines digitalisierten vibroakustischen Signals;
(I) Signalvorverarbeitung des bekannten vibroakustischen Signals (5a);
(II) Identifizierung von Segmenten/Regionen von Interesse (5b); und
(III) Klassifizierung (5e) von Tastereignissen in eine der spezifizierten Klassen, insbesondere in eine Weichheits-/Härteklasse.

Optional erfolgt in einem Schritt (IV) die Ausgabe der Klassifizierung durch eine Ausgabevorrichtung 6 in haptischer und/oder akustischer und/oder optischer Form.

Die Nummerierung der Prozessschritte bezieht sich auf Figur 14.

Die Idee der Erfindung besteht somit insbesondere darin, vibroakustische Signale zu erfassen und in verschiedene Merkmale zu zerlegen. Diese Merkmale können dann mit spezifischen Gewebe-/Oberflächenweichheits-/Härteklassen verknüpft werden.

Entsprechend umfasst das Verfahren zur Merkmalsextraktion und Klassifizierung bei der Palpation die folgenden Schritte:
(0) Bereitstellen eines digitalisierten vibroakustischen Signals;
(I) Signalvorverarbeitung des bekannten vibroakustischen Signals (5a, umfassend die Prozessschritte
   i. Entfernung kontinuierlicher Komponenten (DC) aus dem VA-Signal (5a1);
   ii. Reduzierung des Hintergrundrauschens (5a2);
   iii. Signalverbesserung (5a3);
(II) Identifizierung von Segmenten/Regionen von Interesse (5b); und
(III) Klassifizierung der bei der Palpation entstehenden vibroakustischen Signalen in eine der spezifizierten Klassen, insbesondere ein Weichheits-/Härteklasse (5e), umfassend die Prozessschritte
   i. Identifizierung transienter und stationärer Dynamik (5e1);
   ii. Spektralbandzerlegung (5e2);
   iii. Berechnung von Zeitbereichsindikatoren (5e3), nämlich:
      1. Statistische Indikatoren (5e3_{I});
      2. Morphologische Indikatoren (5e3_{II});
      3. Auf plötzlichen Änderungen basierende Indikatoren (5e3_{III});
   iv. Zeitbereichsmerkmale (5e6), nämlich:
      1. Statistische Indikatoren (5e6_{I});
      2. Morphologische Indikatoren (5e6_{II});
      3. Auf plötzlichen Änderungen basierende Indikatoren (5e6_{III});
   v. Indikatoren im Frequenz- und Zeit-Frequenz-Bereich (5e4), nämlich:
      1. Identifizierung spektrumbasierter Indikatoren (5e4_{I});
      2. Indikatoren auf der Grundlage der momentanen dominanten Frequenz (5e4_{II}); und
   vi. Spektralbereich-Merkmale (5e7), nämlich:
      1. Zeit-Frequenz-Spektrum-Ereignis-Merkmale (5e7_{II});
      2. Statistische Merkmale der momentanen dominanten Frequenz (5e7_{III});
   vii. Klassifizierung durch ein Machine-Learning-Modell (5e10).

Optional erfolgt in einem Schritt (IV) die Ausgabe der Klassifizierung durch eine Ausgabevorrichtung 6 in haptischer und/oder akustischer und/oder optischer Form.

Die Nummerierung der Prozessschritte bezieht sich auf Figur 14.

Das Konzept der *dominanten Frequenz* wird in der Literatur lediglich für stationäre Spektralanalysen (beispielsweise auf der Grundlage der FFT) verwendet, bei denen die Zeit nicht berücksichtigt werden muss. In der Musik- und Sprachsignalverarbeitung ist ein ähnliches Konzept die Tonhöhe. Die Tonhöhe bezieht sich jedoch auf eine "Grundperiode" des Signals, und diese bezeichnet Signale, die recht stationär sind (die spektralen Eigenschaften ändern sich nicht drastisch mit der Zeit). Im vorliegenden Fall wird aufgrund der ausgeprägten nichtstationarität der Signale zu jedem Zeitpunkt eine Schätzung der Frequenzkomponente durchgeführt, die gegenüber den anderen dominiert. Durch diese Erweiterung des Konzepts zur *momentanen dominanten Frequenz* 5e7_{III}, können damit Dynamiken und damit Informationen aus transienten oder hochgradig nicht-stationären Signalen gewonnen werden.

Der Verfahrensschritt, der für die Berechnung der *momentanen dominanten Frequenz* beispielsweise bei der Palpation vorgeschlagen wird, ist im Stand der Technik nicht bekannt.

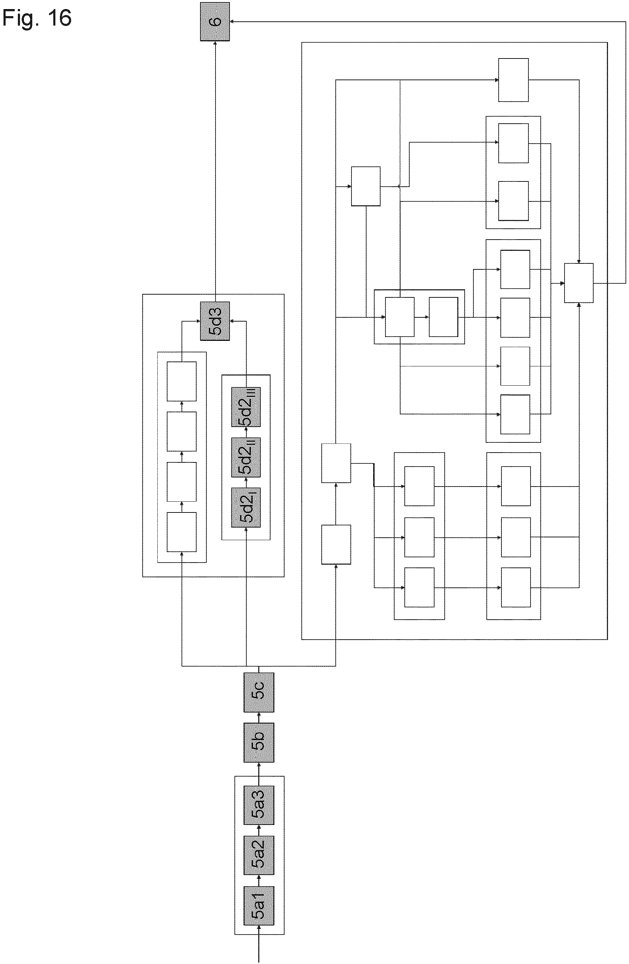

Figur 16 zeigt das Flussdiagramm für die Datenverarbeitung für eine spezifische Anwendung zur indirekten Prüfung einer Nadelpunktion, beispielsweise mit einer Biopsie-Nadel, einer Veres-Nadel oder einer sonstigen Nadel.

Ein bevorzugtes computerimplementiertes Verfahren zur indirekten Audifikation für die indirekten Prüfung einer Nadelpunktion, umfasst die folgenden Schritte:
(0) Bereitstellen eines digitalisierten vibroakustischen Signals;
(I) Signalvorverarbeitung des bekannten vibroakustischen Signals (5a), umfassend die Prozessschritte
   i. Entfernung kontinuierlicher Komponenten (DC) aus dem VA-Signal (5a1);
   ii. Reduzierung des Hintergrundrauschens (5a2);
   iii. Signalverbesserung (5a3);
(II) Identifizierung von Segmenten/Regionen von Interesse (5b); und
(III) Indirekte Audifikation (5d2), umfassend die Prozessschritte
   i. Merkmalsextraktion (5d2_{I});
   ii. Mapping der extrahierten Merkmale (5d2_{II});
   iii. Erzeugung von Klangeigenschaften (5d2_{III}); und
(IV) Übertragung (5d3) an die Ausgabevorrichtung (6).

Die Nummerierung der Prozessschritte bezieht sich auf Figur 14.

Hierbei handelt es sich um ein spezifisches Beispiel für einen Verarbeitungsalgorithmus zur Erzeugung eines akustischen Alarmsignals bei jedem wichtigen Ereignis während des Einführens beispielsweise einer Veres-Nadel. Wenn eine Veres-Nadel in den Bauch eingeführt wird, muss der Chirurg in der Regel drei Hauptebenen durchqueren, um das Ziel, die Bauchhöhle, zu erreichen. Daher sind die drei zu hörenden Ereignisse die folgenden:
- Ereignis 1: Gewebe-Gewebe-Grenze 1 (erste fascia) Punktion
- Ereignis 2: Gewebe-Gewebe-Grenze 2 (zweite fascia) Punktion
- Ereignis 3: Punktion der Zielstruktur (Peritoneum).

Bei anderen Aufgaben einer Nadelpunktion können auch weniger oder mehr Hauptebenen zu durchqueren sein, hieran kann das erfinderische Verfahren einfach angepasst werden.

Die grauen Blöcke stellen aktive Verarbeitungsblöcke für die gegebene spezifische Anwendung dar mit den Schritten 5a1, 5a2, 5a3, 5b, 5c, 5d2_{I}, 5d2_{II}, 5d2_{III}, 5d3 sowie die Weiterleitung an die Ausgabevorrichtung 6. Die Ausgabevorrichtung 6 kann derart vorgesehen sein, dass sie bei jedem Ereignis eine spezifische Ausgabe in optischer, akustischer oder haptischer Art generiert, sodass der Anwender über den Fortschritt der Punktion jederzeit unterrichtet ist. Die Benennung der Verfahrensschritte bezieht sich auf die Figur 14.

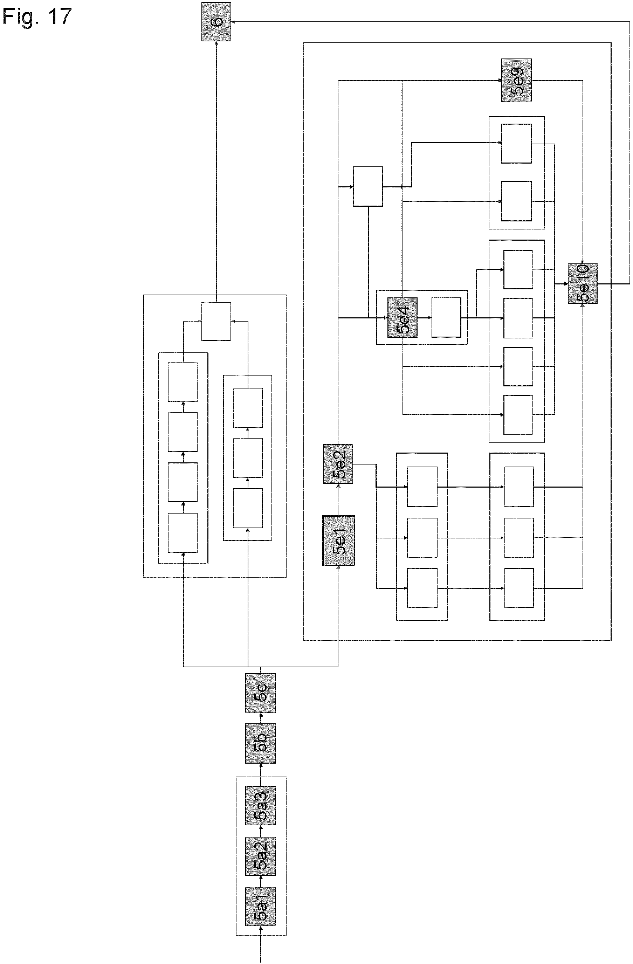

Figur 17 zeigt das Flussdiagramm für die spezifische Verarbeitung für die automatische Erkennung von laparoskopischen Instrumenten, wie z. B. in den Konfigurationen in den Figuren 11, 12 und 13.

Ein bevorzugtes computerimplementiertes Verfahren zur automatischen Erkennung von laparoskopischen Instrumenten, umfasst die folgenden Schritte:
(0) Bereitstellen eines digitalisierten vibroakustischen Signals;
(I) Signalvorverarbeitung des bekannten vibroakustischen Signals (5a), umfassend die Prozessschritte:
   i. Entfernung kontinuierlicher Komponenten (DC) aus dem VA-Signal (5a1);
   ii. Reduzierung des Hintergrundrauschens (5a2);
   iii. Signalverbesserung (5a3);
(II) Identifizierung von Segmenten/Regionen von Interesse (5b); und
(III) Klassifizierung (5e) des laparoskopischen Werkzeugs, umfassend die Prozessschritte:
   i. Spektralbandzerlegung (5e2);
   ii. Identifizierung spektrumbasierter Indikatoren (5e4_{I});
   iii. Einspeisen der Daten in einen Deep-Learning-Algorithmus (5e9); und
   iv. Klassifizierung durch ein Machine-Learning-Modell (5e10);

Optional erfolgt in einem Schritt (IV) die Speicherung der Klassifizierung in der Ausgabevorrichtung (6).

Die Nummerierung der Prozessschritte bezieht sich auf Figur 14.

Wenn ein laparoskopisches Werkzeug durch den Trokar eingeführt wird, führt die erzeugte Interaktion (innerer Teil des Trokars mit dem Instrument) zu einem VA-Signal, das zur Klassifizierung der Art des in dem Operationsprozess verwendeten laparoskopisches Werkzeugs genutzt werden kann. Da die Signatur des VA-Signals sehr komplex sein kann, wird die Merkmalsextraktion nach den Schritten 5a1, 5a2, 5a3, 5b, 5c, 5e2, 5e4_{I} weiterhin mit Methoden des Deep Learning 5e9 durchgeführt. Die Merkmale werden in ein trainiertes Machine-Learning-Modell 5e10 eingespeist, das das Instrument identifiziert. Diese Informationen können für eine spätere Verwendung in der Ausgabevorrichtung 6 gespeichert oder auch zur Erstellung automatischer Operationsberichte verwendet werden. Die Benennung der Verfahrensschritte bezieht sich auf die Figur 14.

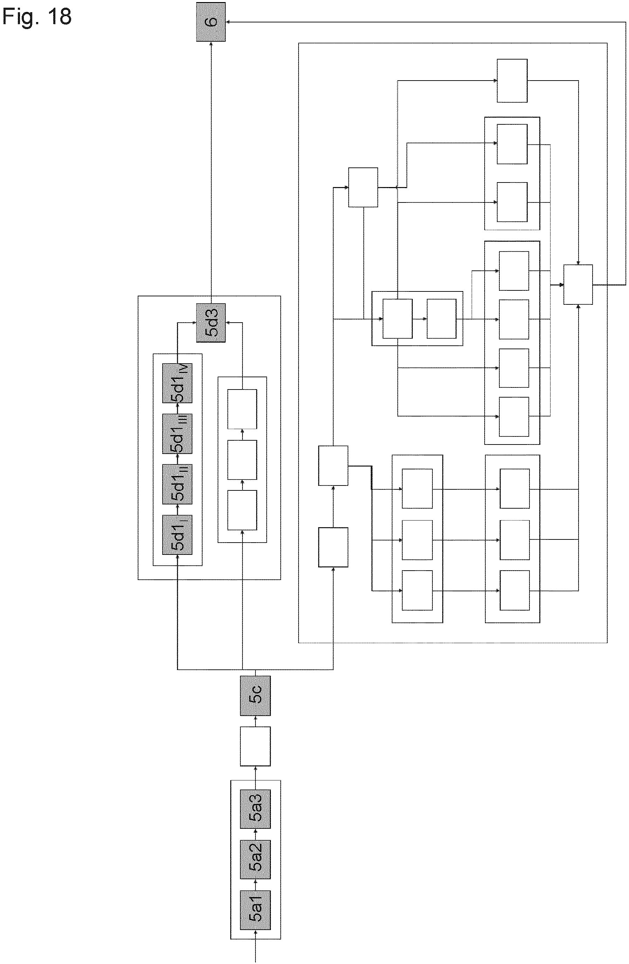

Figur 18 zeigt das Flussdiagramm für die spezifische Datenverarbeitung einer Audifikation bei laparoskopischen Eingriffen gemäß Figur 11.

Ein bevorzugtes computerimplementiertes Verfahren zur direkten Audifikation bei laparoskopischen Eingriffen, umfasst die folgenden Schritte:
(0) Bereitstellen eines digitalisierten vibroakustischen Signals;
(I) Signalvorverarbeitung des bekannten vibroakustischen Signals (5a), umfassend die Prozessschritte:
   i. Entfernung kontinuierlicher Komponenten (DC) aus dem VA-Signal (5a1);
   ii. Reduzierung des Hintergrundrauschens (5a2);
   iii. Signalverbesserung (5a3);
(II) Identifizierung von Segmenten/Regionen von Interesse (5b); und
(III) Direkte Audifikation (5d1), umfassend die Prozessschritte:
   i. Filterung des Signals (5d1_{I)});
   ii. Anpassung der Lautstärke (5d1_{II});
   iii. Bearbeitung der Dynamik (5d1_{III,});
   iv. Erzeugung von Effekten (5d1 iv);
(IV) Übertragung (5d3) an die Ausgabevorrichtung (6).

Die Nummerierung der Prozessschritte bezieht sich auf Figur 14.

Das Hauptziel der laparoskopischen Audifikation ist es, dem tonlosen Videoendoskop mehr Transparenz zu verleihen, indem jede Werkzeug-Gewebe-Interaktion der laparoskopischen Werkzeuge, die an einem bestimmten Eingriff beteiligt sind, hervorgehoben und auditiert wird. Dies kann in Echtzeit zur Verbesserung der Operationswahrnehmung während einer realen Operation oder für die Ausbildung von Chirurgen genutzt werden. Es kann ein direkter Auditionsalgorithmus verwendet werden, bei dem die Interaktionen jedes an der Operation beteiligten Werkzeugs parallel verarbeitet wird und bei dem jedes Werkzeug über verschiedene Lautsprecher wiedergegeben werden kann.

Figur 19 zeigt das Flussdiagramm der Datenverarbeitung einer spezifischen Anwendung zur vibroakustischen Echtzeit-Überwachung während elektrochirurgischer Eingriffe zur Charakterisierung von unterschiedlichen Geweben beziehungsweise unterschiedlichen Gewebszuständen.

Elektrochirurgie und Elektrokauterisation spielen in der minimalinvasiven Chirurgie, insbesondere in der laparoskopischen und robotergestützten Chirurgie, eine wesentliche Rolle. Dabei wird elektrische Energie verwendet, um Gewebe zu erhitzen und so chirurgische Aufgaben wie Schneiden oder Koagulieren zur Blutstillung durchzuführen. Diese Verfahren sind für die präzise Behandlung von Gewebe bei minimalinvasiven Eingriffen von entscheidender Bedeutung.

Trotz ihrer Nützlichkeit sind elektrochirurgische Verfahren mit Herausforderungen verbunden, darunter das Risiko von Verbrennungen und unbeabsichtigten Gewebeschäden. Es besteht ein entscheidender Bedarf an Technologien, die Gewebeveränderungen während elektrochirurgischer Eingriffe in Echtzeit überwachen können. Dieses Feedback ist für Chirurgen unerlässlich, um die genannten Komplikationen zu vermeiden. Um diesen Bedarf zu decken, stellt die Erfindung nicht-invasive Überwachungsmethoden auch in Echtzeit bereit. Die Identifizierung von Gewebezuständen beziehungsweise -phasen während der Erwärmung ist von entscheidender Bedeutung, um die Energieabgabe der medizinischen Geräte zu optimieren und nachteilige Auswirkungen zu vermeiden.

Die vorliegende Erfindung kann für die Überwachung während dieser Art von Verfahren eingesetzt werden. Das vibroakustische Sensormodul, das an das proximale Ende elektrochirurgischer Instrumente angeschlossen werden kann (die meisten entsprechen in ihrem Aussehen Standard-Laparoskopie-Instrumenten), ermöglicht die Echtzeit-Erfassung vibroakustischer Wellen während des Eingriffs. Da sich die Gewebemerkmale mit den durch die elektrische Erwärmung erzeugten Gewebeeffekten verändern, können diese Veränderungen durch die vibroakustischen Signale erfasst und erkannt werden. Die Erfindung kann diese Signale interpretieren und ermöglicht dem Chirurgen die Identifizierung und Verfolgung von Gewebeveränderungen auch in Echtzeit. Durch die Umwandlung dieser Signale in Informationen zur Überwachung der Auswirkungen der Erwärmung auf das Gewebe erhalten die Chirurgen Erkenntnisse, um die Energieabgabe während der Aktivierung des elektrochirurgischen Geräts und den Zeitpunkt der Exposition des elektrochirurgischen Geräts anzupassen.

Im praktischen Einsatz dient die Erfindung als Zusatzgerät, das an elektrochirurgische Instrumente angeschlossen wird, wie in Figur 11 dargestellt. Während des elektrochirurgischen Eingriffs erfasst die Kombination aus Sensoreinheit und Verarbeitungseinheit Veränderungen der Gewebemerkmale und ermöglicht die Identifizierung und Verfolgung in Echtzeit. Chirurgen erhalten sofortige Rückmeldung über Gewebeveränderungen und können so während des Eingriffs fundierte Anpassungen vornehmen. Diese Fähigkeit macht die Erfindung zu einem wertvollen Instrument zur Erhöhung der Präzision, zur Reduzierung von Komplikationen und zur Verbesserung der Patientenergebnisse in der Elektrochirurgie.

Viele der elektrochirurgischen Werkzeuge haben die gleichen Eigenschaften wie die Standard-Laparoskopie-Werkzeuge. Daher ist die Ausführungsform gemäß Abbildung 11 auch für diesen Anwendungsfall geeignet, da das Sensormodul über einen Adapter im Schaft oder indirekt über den Luer-Lock des Trokars an das elektrochirurgische Instrument angeschlossen werden kann. Das vibroakustische Signal wird durch das Sensormodul während des Erhitzungsprozesses erfasst. Das Signal wird drahtlos oder kabelgebunden an eine Haupteinheit übertragen, die das Signal verarbeitet und die vibroakustischen Muster entsprechend der Erwärmungsstufe des Gewebes klassifiziert.

Figur 19 zeigt grau unterlegt die Blöcke, die für das erfindungsgemäße Verfahren im Zusammenhang mit der Elektrochirurgie aus dem allgemeinen Verarbeitungsdiagramm von Figur 14 verwendet werden.

Die grundsätzliche Idee der Erfindung, vibroakustische Signale zu erfassen und in verschiedene Merkmale zu zerlegen, wird entsprechend auch in diesem Zusammenhang angewendet. Die Merkmale können dann mit spezifischen Gewebeeigenschaften verknüpft werden.

Entsprechend umfasst das Verfahren zur Merkmalsextraktion und Klassifizierung bei der Elektrochirurgie die folgenden Schritte:
(0) Bereitstellen eines digitalisierten vibroakustischen Signals;
(I) Signalvorverarbeitung des bekannten vibroakustischen Signals (5a, umfassend die Prozessschritte
   i. Entfernung kontinuierlicher Komponenten (DC) aus dem VA-Signal (5a1);
   ii. Reduzierung des Hintergrundrauschens (5a2);
   iii. Signalverbesserung (5a3);
(II) Klassifizierung der bei der Elektrochirurgie entstehenden vibroakustischen Signalen in eine der spezifizierten Klassen, insbesondere eine Gewebewärmestufen (5e), umfassend die Prozessschritte
   i. Identifizierung transienter und stationärer Dynamik (5e1);
   ii. Spektralbandzerlegung (5e2);
   iii. Indikatoren im Frequenz- und Zeit-Frequenz-Bereich (5e4), nämlich:
      1. Identifizierung spektrumbasierter Indikatoren (5e4_{I});
      2. Indikatoren auf der Grundlage der momentanen dominanten Frequenz (5e4_{II}); und
   iv. Spektralbereich-Merkmale (5e7), nämlich:
      1. Frequenz-Spektrum-Ereignis-Merkmale (5e7_{I});
      2. Zeit-Frequenz-Spektrum-Ereignis-Merkmale (5e7_{II});
      3. Statistische Merkmale der momentanen dominanten Frequenz (5e7_{III});
      4. Dynamische Merkmale der momentanen dominanten Frequenz 5e7_{IV};
   v. Klassifizierung durch ein Machine-Learning-Modell (5e10).

Optional erfolgt in einem Schritt (IV) die Ausgabe der Klassifizierung durch eine Ausgabevorrichtung 6 in haptischer und/oder akustischer und/oder optischer Form.

Die Nummerierung der Prozessschritte bezieht sich auf Figur 14.

Bei der Datenverarbeitung (Schritt 5e10) wird das vibroakustische Signal, das sich aus den verschiedenen Gewebewärmestufen ergibt, insbesondere in die folgenden Klassen eingeteilt:
Klasse 1: Hyperthermie (ab 40°): initiale Gewebeschädigung, Ödembildung, je nach Dauer der Anwendung kann sich das Gewebe erholen oder absterben (Devitalisierung).
Klasse2: Devitalisierung (Zerstörung) der Zellen, Schrumpfung des Bindegewebes durch Denaturierung (ab 60°)
Klasse 3: Verdampfung der Gewebeflüssigkeit, je nach Geschwindigkeit der Verdampfung (um 100°):
Klasse 4: Verkohlung (ab ca. 150°)

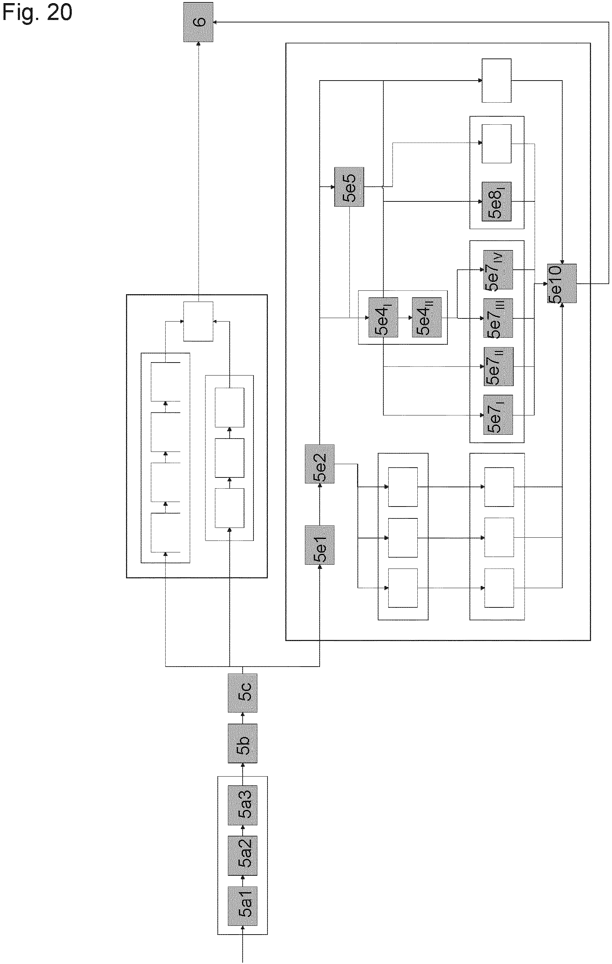

Figur 20 zeigt das Flussdiagramm für die Datenverarbeitung für eine spezifische Anwendung zur vibroakustischen Echtzeit-Überwachung für präzise Knochenbohrungen oder Verschraubungen (nachfolgend werden "Bohrungen" und "Verschraubungen" vereinfachend als "Bohrungen" oder "Knochenbohrungen" zusammengefasst) bei chirurgischen Eingriffen.

Das Bohren von Knochen ist in verschiedenen chirurgischen Disziplinen wie der orthopädischen Chirurgie, der Ohrchirurgie, der Kiefer- und Gesichtschirurgie und der Neurochirurgie erforderlich. Bei diesen Eingriffen besteht die Herausforderung darin, dass das Bohren von Knochen manuell erfolgt und die Chirurgen oft Schwierigkeiten haben, die richtige Menge an Material zu entnehmen oder die gewünschte Tiefe zu erreichen. Die Trägheit der Bohrkraft und physiologische Erschütterungen können zu unbeabsichtigten Schäden führen, beispielsweise kann ein Bohrer während eines Durchbruchs weiter als vorgesehen vorgeschoben werden. Die derzeitige Abhängigkeit einer erfolgreichen Intervention von der Erfahrung und Intuition des Chirurgen macht den Bedarf an einer nicht-invasiven und objektiven Überwachungslösung deutlich.

Das händisch durchgeführte Knochenbohren stellt eine Herausforderung für den Chirurgen dar, da es schwierig ist, die Intervention und insbesondere die Bohrung präzise zu kontrollieren. Auch die elektronische Überwachung des Knochenbohrens ist schwierig, da es keine unmittelbare Rückmeldung gibt, insbesondere wenn Durchbrüche auftreten. Die derzeitigen Überwachungsmethoden, wie beispielsweise Kraftmessung und Schallemission, haben ihre eigenen Probleme. Die Kraftsensorik reagiert nur langsam auf schnelle Veränderungen während des Bohrens und hat eine begrenzte Reichweite. Die Schallemission reagiert empfindlich auf den Lärm, der durch den Bohrvorgang selbst entsteht.

Die Erfindung geht auf den ungedeckten klinischen Bedarf an nicht-invasiver Überwachung während des Knochenbohrens ein. Im Gegensatz zu herkömmlichen Methoden erfasst die Erfindung vibroakustische Wellen, deren Dynamik in direktem Zusammenhang mit Dichteänderungen, einschließlich Knochenbrüchen, steht. Dieser Ansatz ermöglicht eine Echtzeitverarbeitung der resultierenden vibroakustischen Wellen zur Überwachung des Knochenbohrprozesses. Die Eigenschaften der vom System erfassten vibroakustischen Wellen ändern ihr Verhalten, wenn sich die Knochendichte ändert. Auf diese Weise ist es möglich, die Dichteänderungen in Echtzeit zu charakterisieren, indem Merkmale aus dem vibroakustischen Signal extrahiert werden.

Zu diesem Zweck kann das Sensormodul, wie in Abbildung 11A dargestellt, über einen Standardanschluss wie beispielsweise ein Bohrfutter oder einen speziell entwickelten Adapter am proximalen Ende des Bohrers angebracht werden. Das vibroakustische Signal wird vom Sensormodul erfasst und drahtlos oder kabelgebunden an eine Haupteinheit übertragen, die das Signal verarbeitet, um eine Echtzeit-Rückmeldung zu liefern, wenn der Bohrer von einer Art von Knochendichte zu einer anderen Art von Knochendichte übergeht oder wenn er den Knochen bricht. Der Signalverarbeitungspfad umfasst hauptsächlich Vorverarbeitung, spektrale und hüllkurvenbasierte Merkmalsextraktion und schließlich ein trainiertes maschinelles Lernmodell, das den Zeitpunkt erkennt, an dem sich die Knochendichte ändert und ein Knochendurchbruch erfolgt. In dem Moment, in dem der Algorithmus diese Veränderungen erkennt, gibt er dem Benutzer eine Rückmeldung.

Entsprechend umfasst das Verfahren für präzise Knochenbohrungen bei chirurgischen Eingriffen die folgenden Schritte:
(0) Bereitstellen eines digitalisierten vibroakustischen Signals;
(I) Signalvorverarbeitung des bekannten vibroakustischen Signals (5a, umfassend die Prozessschritte
   i. Entfernung kontinuierlicher Komponenten (DC) aus dem VA-Signal (5a1);
   ii. Reduzierung des Hintergrundrauschens (5a2);
   iii. Signalverbesserung (5a3);
(II) Identifizierung von Segmenten/Regionen von Interesse (5b);
(III) Klassifizierung der bei der Bohrung entstehenden vibroakustischen Signalen in eine der spezifizierten Klassen, insbesondere eine Knochenschichtsklasse (5e, siehe Figur 11A), umfassend die Prozessschritte
   i. Identifizierung transienter und stationärer Dynamik (5e1);
   ii. Spektralbandzerlegung (5e2);
   iii. Indikatoren im Frequenz- und Zeit-Frequenz-Bereich (5e4), nämlich:
      1. Identifizierung spektrumbasierter Indikatoren (5e4_{I});
      2. Indikatoren auf der Grundlage der momentanen dominanten Frequenz (5e4_{II}); und
   iv. Hüllkurvenbasierte Indikatoren 5e5;
   v. Spektralbereich-Merkmale (5e7), nämlich:
      1. Frequenz-Spektrum-Ereignis-Merkmale (5e7_{I});
      2. Zeit-Frequenz-Spektrum-Ereignis-Merkmale (5e7_{II});
      3. Statistische Merkmale der momentanen dominanten Frequenz (5e7_{III});
      4. Dynamische Merkmale der momentanen dominanten Frequenz 5e7_{IV};
   vi. Spektrale Hüllkurvenmerkmale 5e8_{I};
   vii. Klassifizierung durch ein Machine-Learning-Modell (5e10).

Optional erfolgt in einem Schritt (IV) die Ausgabe der Klassifizierung durch eine Ausgabevorrichtung 6 in haptischer und/oder akustischer und/oder optischer Form und zeigt dem Anwender beispielsweise eine Veränderung der Knochendichte an.

Die Nummerierung der Prozessschritte bezieht sich auf Figur 14.

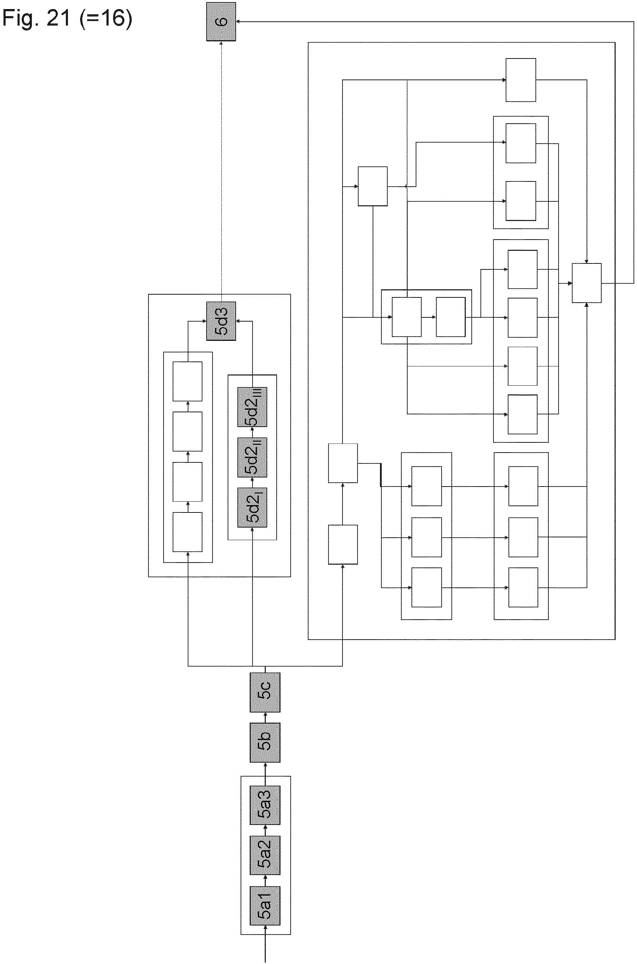

Figur 21 zeigt das Flussdiagramm für die Datenverarbeitung einer spezifischen Anwendung zur Erkennung und Vorbeugung von Schäden an pulsierenden Strukturen in der laparoskopischen und robotergestützten Chirurgie.

Bei robotergestützten und laparoskopischen Eingriffen ist die präzise Erkennung von pulsierenden Strukturen, wie beispielsweise von Blutgefäßen, von entscheidender Bedeutung, um versehentliche Schäden zu vermeiden. Derzeit stützt sich die Wahrnehmung dieser Strukturen durch den Chirurgen auf visuelle Hinweise und individuelle Erfahrung. Die Herausforderung bei der Erkennung solcher Strukturen liegt in Situationen, in denen pulsierende Strukturen während des Eingriffs nicht sofort erkennbar oder sichtbar sind. Es besteht somit ein klinischer Bedarf an Technologien, die diese pulsierenden Strukturen in Echtzeit erkennen können, um die Sicherheit dieser Eingriffe zu erhöhen, indem dem Chirurgen in Echtzeit Rückmeldung über die Nähe einer pulsierenden Struktur gegeben und so das Risiko einer unbeabsichtigten Beschädigung verringert wird. Die Information über das Vorhandensein einer pulsierenden Struktur kann alternativ auch an ein Robotersystem weitergeleitet werden, wenn der Chirurg eine pulsierende Struktur in der Nähe nicht bemerkt. Dieser erfinderische Ansatz stellt sicher, dass das Robotersystem selbst dann, wenn der Chirurg eine potenziell schädliche Aktion einleitet, deren Ausführung verhindern kann.

Um dieses Problem zu lösen, kann die Erfindung automatisch und in Echtzeit das Vorhandensein dieser pulsierenden Strukturen erkennen und dem Chirurgen und/oder dem Roboter eine direkte Rückmeldung geben.

Wenn die Spitze eines laparoskopischen oder robotergestützten Instruments in direkten Kontakt mit einem Gefäß oder dem umgebenden Binde- oder Deckgewebe kommt, pulsiert das Gefäß und verursacht Bewegungen auch im umgebenden Gewebe. Diese Interaktion erzeugt Vibrationen an der Instrumentenspitze, die sich durch das Instrument ausbreiten und vom Sensormodul erfasst werden. Dieses vibroakustische Signal wird dann entweder drahtlos oder über ein Kabel an eine zentrale Verarbeitungseinheit übertragen. Diese Einheit verarbeitet das Signal und ermöglicht es, das Vorhandensein einer pulsierenden Struktur in der Nähe zu erkennen und den Benutzer über dieses Ereignis zu informieren.

Entsprechende Ausführungsformen der Erfindung finden sich auch in den Figuren 11 und 11B.

Die Datenverarbeitung umfasst, wie in dem Flussdiagramm dargestellt, in erster Linie eine Vorverarbeitungsstufe zur Reduzierung von Hintergrundrauschen und zur Verbesserung der periodischen Wechselwirkungen im Zusammenhang mit der Pulsation. Anschließend werden die Pulsationen identifiziert und ihre Intensität in eine periodische akustische Rückkopplung mit Nebengeräuschen übersetzt, die sowohl mit der Intensität als auch mit der Frequenz der Pulsation korreliert. Je näher sich das Instrument an der pulsierenden Struktur befindet, desto höher ist die Intensität des periodischen akustischen Feedbacks. Auf diese Weise kann der Benutzer die Nähe zu einer pulsierenden Struktur beurteilen - keine akustische Rückmeldung bedeutet, dass keine pulsierende Struktur in der Nähe ist.

Die grauen Blöcke stellen aktive Verarbeitungsblöcke für die gegebene spezifische Anwendung dar mit den Schritten 5a1, 5a2, 5a3, 5b, 5c, 5d2_{I}, 5d2_{II}, 5d2_{III}, 5d3 sowie die Weiterleitung an die Ausgabevorrichtung 6. Die Ausgabevorrichtung 6 kann derart vorgesehen sein, dass sie bei jedem Ereignis eine spezifische Ausgabe in optischer, akustischer oder haptischer Art generiert, sodass der Anwender über die Nähe zu einem pulsierenden Objekt jederzeit unterrichtet ist.

Ein bevorzugtes computerimplementiertes Verfahren zur Erkennung und Vorbeugung von Schäden an pulsierenden Strukturen in der laparoskopischen und robotergestützten Chirurgie, umfasst die folgenden Schritte:
(0) Bereitstellen eines digitalisierten vibroakustischen Signals;
(I) Signalvorverarbeitung des bekannten vibroakustischen Signals (5a), umfassend die Prozessschritte
   i. Entfernung kontinuierlicher Komponenten (DC) aus dem VA-Signal (5a1);
   ii. Reduzierung des Hintergrundrauschens (5a2);
   iii. Signalverbesserung (5a3);
(II) Identifizierung von Segmenten/Regionen von Interesse (5b); und
(III) Indirekte Audifikation (5d2), umfassend die Prozessschritte
   i. Merkmalsextraktion (5d2_{I});
   ii. Mapping der extrahierten Merkmale (5d2_{II});
   iii. Erzeugung von Klangeigenschaften (5d2_{III}); und
(IV) Übertragung (5d3) an die Ausgabevorrichtung (6).

Die Nummerierung der Prozessschritte bezieht sich auf Figur 14.

Figur 22 zeigt das Flussdiagramm für die Datenverarbeitung für eine spezifische Anwendung zur Echtzeit-Erkennung und Klassifizierung von Kollisionen bei robotergestützten Operationen mit der erfindungsgemäßen Technologie.

Bei robotergestützten Operationen kann das Fehlen einer haptischen Rückmeldung zu unerkannten Kollisionen führen, die Verzögerungen und potenzielle Schäden verursachen. Fälle wie sich gegenseitig blockierende Roboterarme oder Kollisionen zwischen laparoskopischen Instrumenten bleiben vom Chirurgen oft unbemerkt und verlängern die Operationszeit. Noch kritischer wird es, wenn das Werkzeug des Roboters mit Weichteilgewebe kollidiert, was zu Schäden führen kann, die der Chirurg nicht sofort erkennt. Die erfindungsgemäße Technologie stellt eine Lösung bereit.

Das Sensormodul erfasst vibroakustische Signale, die durch verschiedene Kollisionen erzeugt werden - seien es Kollisionen von Instrumenten, Blockaden des Roboterarms oder der Kontakt mit Weichgewebe. Die unterschiedlichen Charakteristika dieser vibroakustischen Signale ermöglichen es, Kollisionen in Echtzeit zu identifizieren und zu klassifizieren und den Chirurgen eine unmittelbare Rückmeldung zu geben und so zur Sicherheit und Effizienz robotergestützter Operationen beizutragen. Details hierzu finden sich auch in Figur 11B.

Entsprechend umfasst das Verfahren zur Merkmalsextraktion und Klassifizierung zur Echtzeit-Erkennung und Klassifizierung von Kollisionen bei robotergestützten Operationen mit der erfindungsgemäßen Technologie die folgenden Schritte:
(0) Bereitstellen eines digitalisierten vibroakustischen Signals;
(I) Signalvorverarbeitung des bekannten vibroakustischen Signals (5a, umfassend die Prozessschritte
   i. Entfernung kontinuierlicher Komponenten (DC) aus dem VA-Signal (5a1);
   ii. Reduzierung des Hintergrundrauschens (5a2);
   iii. Signalverbesserung (5a3);
(II) Identifizierung von Segmenten/Regionen von Interesse (5b); und
(III) Klassifizierung der bei robotergestützten Operationen entstehenden vibroakustischen Signalen in eine der spezifizierten Klassen, insbesondere eine Kollisionsklasse:
   i. Identifizierung transienter und stationärer Dynamik (5e1);
   ii. Spektralbandzerlegung (5e2);
   iii. Berechnung von Zeitbereichsindikatoren (5e3), nämlich:
      1. Statistische Indikatoren (5e3_{I});
      2. Morphologische Indikatoren (5e3_{II});
      3. Auf plötzlichen Änderungen basierende Indikatoren (5e3_{III});
   iv. Zeitbereichsmerkmale (5e6), nämlich:
      1. Statistische Indikatoren (5e6_{I});
      2. Morphologische Indikatoren (5e6_{II});
      3. Auf plötzlichen Änderungen basierende Indikatoren (5e6_{III});
   v. Indikatoren im Frequenz- und Zeit-Frequenz-Bereich (5e4), nämlich:
      1. Identifizierung spektrumbasierter Indikatoren (5e4_{I});
      2. Indikatoren auf der Grundlage der momentanen dominanten Frequenz (5e4_{II}); und
   vi. Spektralbereich-Merkmale (5e7), nämlich:
      1. Frequenz-Spektrum-Ereignis-Merkmale (5e7_{I});
      2. Zeit-Frequenz-Spektrum-Ereignis-Merkmale (5e7_{II});
      3. Statistische Merkmale der momentanen dominanten Frequenz (5e7_{III});
      4. Dynamische Merkmale der momentanen dominanten Frequenz 5e7_{IV};
   vii. Klassifizierung durch ein Machine-Learning-Modell (5e10).

Optional erfolgt in einem Schritt (IV) die Ausgabe der Klassifizierung durch eine Ausgabevorrichtung 6 in haptischer und/oder akustischer und/oder optischer Form.

Die Nummerierung der Prozessschritte bezieht sich auf Figur 14.

Ein oder mehrere Sensormodule werden an das proximale Ende von Roboterarmen angeschlossen, deren Instrumente eine Kollisionsüberwachung erfordern. Das Sensormodul erfasst vibroakustische Signale und überträgt sie drahtlos oder über Kabel an eine Haupteinheit zur Signalverarbeitung. Der in der Figur 22 dargestellte Signalverarbeitungspfad umfasst insbesondere eine Vorverarbeitungsstufe zur Reduzierung von Hintergrundgeräuschen und zur Verbesserung der Interaktion. Anschließend werden mögliche Kollisionsereignisse (Region of Interest) identifiziert, und aus jedem Ereignis werden Merkmale extrahiert, um festzustellen, ob es sich um eine Kollision handelt. Wenn eine Kollision bestätigt wird, klassifiziert das zu diesem Zweck trainierte maschinelle Lernmodell die Art der Kollision. Die Klassifizierung umfasst:
Klasse 1: Keine Kollision
Klasse 2: Kollision zwischen Instrument und Instrument
Klasse 3: Kollision mit dem Roboterarm
Klasse 4: Kollision zwischen Instrument und Weichgewebe

### Bezuaszeichenliste

- 1: Interaktives Gewebe
- 1a: Oberfläche des Gewebes
- 1b: Ziel/anatomische Struktur im Gewebe
- 1c: Ziel/anatomische Struktur, die von Gewebe bedeckt ist
- 1d: Ziel/anatomische Struktur im Hohlraum unter dem Gewebe
- 1e: Gewebe-Gewebe-Grenze
- 1f: Rand der Gewebekavität
- 2: Medizinisches Instrument (2a: Trokar; 2b: Laparoskop; 2c: Bohrer, Knochenschraube)
- 3: Adapterelement
- 4: Signalerfassungsvorrichtung
- 4a: Kopplungselement
- 4b: Mechanischer Verstärkungsmechanismus
- 4c: Akustik- oder Vibrationssensor
- 4d: Analoge Signalvorverarbeitung
- 4e: Analog-Digital-Wandlung
- 4f: Controller
- 4g: Kommunikationseinheit
- 5: Daten-/ Signalverarbeitungsvorrichtung
- 6: Ausgabevorrichtung
- 6a: Lautsprecher
- 6b: Speicher/Datenbank
- 6c: VR/AR-Gerät
- 6d: Bildschirm zur Visualisierung
- 6e: Haptisches Gerät
- 6f: Bericht
- 7: Körper
- 8: Endoskopisches Kamerasystem
- 8a: Kamera
- 8b: Endoskop
- 8c: Sichtfeld (FOV)
- 8d: Sichtlinie (LOS)
- 9: Robotische Instrument
- 10: Chirurgischer Roboter/Roboterarm
- 11: Chirurgische Bohrmaschine
- 12: Knochen
- 12a: Knochenschicht mit der Dichte a
- 12b: Knochenschicht mit der Dichte b
- 12c: Knochenschicht mit der Dichte c
- 12d: letzte Knochenschicht vor dem Durchbohren
- 13: Kollision

## Patentansprüche

1. Vorrichtung zum Anschluss an ein medizinisches Instrument zur Erfassung von vibroakustischen Signalen, wobei die Vorrichtung eine Signalerfassungsvorrichtung (4) mit einem Kopplungselement (4a) zum Anschluss eines medizinischen Instruments (2), zumindest einen Sensor (4c) zur Erfassung vibroakustischer Signale, eine Analog-Digital-Wandlung (4e) zur Umwandlung der von dem mindestens einen Sensor erfassten analogen Signale in digitale Daten, einen Controller (4f) und eine Kommunikationseinheit (4g) zum Datenaustausch umfasst.

2. Vorrichtung nach Anspruch 1, **dadurch gekennzeichnet, dass** das Kopplungselement (4a) das medizinische Instrument (2) und den zumindest einen Sensor (4c) schallübertragend verbindet, wobei das Kopplungselement (4a) geeignet ist, vibroakustische Signale möglichst störungs- und verlustfrei von dem Instrument (2) an den mindestens einen Sensor (4c) zu übertragen und wobei der mindestens eine Sensor (4c) insbesondere kraft-, form- oder reibschlüssig direkt mit dem Kopplungselement (4a) verbunden ist.

3. Vorrichtung nach Anspruch 1, **dadurch gekennzeichnet, dass** die Signalerfassungsvorrichtung (4) einen mechanischen Verstärkungsmechanismus (4b) mit einer Membran und einem Schallraum umfasst, wobei das Kopplungselement (4a) geeignet ist, vibroakustische Signale möglichst störungs- und verlustfrei von dem Instrument (2) an die Membran zu übertragen und nicht dauerhaft aber zumindest kraft- oder reibschlüssig, also wirkungsübertragend, mit der Membran verbunden ist, wobei die Membran die vibroakustischen Signale in einen sich an die Membran anschließenden Schallraum überträgt, wobei die vibroakustischen Signale eine Impuls-Komponente aufweisen, die in axialer Richtung, also senkrecht zur Membran, wirkt und die zu einer Anregung beziehungsweise Auslenkung der Membran führt, und ein zusätzliches Biegemoment im Kopplungspunkt des Kopplungselements (4a) mit der Membran aufweist, wobei sowohl durch die axiale Impulskomponente als auch durch das Biegemoment eine Auslenkung der Membran und damit eine Schallwelle beziehungsweise eine Druckwelle in dem sich an die Membran anschließenden Schallraum erzeugt und wobei der mindestens eine Sensor (4c) an dem Schallraum vorgesehen ist und diesem vorzugsweise begrenzt.

4. Computerimplementiertes Verfahren zur Bearbeitung und Auswertung vibroakustischer Signale umfassend folgende Schritte:
(0) Bereitstellen eines vorhandenen digitalisierten vibroakustischen Signals;
(I) Signalvorverarbeitung des vibroakustischen Signals (5a); und
(II) Identifizierung von Segmenten/Regionen von Interesse (5b); und
(III) Audifikation (5d); und/oder (5c)
(IV) Klassifizierung der Segmente/Regionen von Interesse (5e),
wobei optional in einem weiteren Schritt (V) beispielsweise die Ausgabe, Weiterverarbeitung oder Speicherung (6) der klassifizierten Segmente/Regionen in einer Ausgabevorrichtung erfolgt.

5. Computerimplementiertes Verfahren zur Bearbeitung und Auswertung vibroakustischer Signale gemäß Anspruch 4 zur Merkmalsextraktion und Klassifizierung von bei der Palpation entstehenden Signalen umfassend folgende Schritte:
(0) Bereitstellen eines digitalisierten vibroakustischen Signals;
(I) Signalvorverarbeitung des bekannten vibroakustischen Signals (5a), umfassend die Prozessschritte:
i. Entfernung kontinuierlicher Komponenten (DC) aus dem VA-Signal (5a1);
ii. Reduzierung des Hintergrundrauschens (5a2);
iii. Signalverbesserung (5a3);
(II) Identifizierung von Segmenten/Regionen von Interesse (5b); und
(III) Klassifizierung der bei der Palpation entstehenden vibroakustischen Signale in eine der spezifizierten Klassen, insbesondere ein Weichheits-/Härteklasse (5e), umfassend die Prozessschritte:
i. Identifizierung transienter und stationärer Dynamik (5e1);
ii. Spektralbandzerlegung (5e2);
iii. Berechnung von Zeitbereichsindikatoren (5e3), nämlich:
1. Statistische Indikatoren (5e3_{I});
2. Morphologische Indikatoren (5e3_{II});
3. Auf plötzlichen Änderungen basierende Indikatoren (5e3_{III});
iv. Zeitbereichsmerkmale (5e6), nämlich:
1. Statistische Indikatoren (5e6_{I});
2. Morphologische Indikatoren (5e6_{II});
3. Auf plötzlichen Änderungen basierende Indikatoren (5e6_{III});
v. Indikatoren im Frequenz- und Zeit-Frequenz-Bereich (5e4), nämlich:
1. Identifizierung spektrumbasierter Indikatoren (5e4_{I});
2. Indikatoren auf der Grundlage der momentanen dominanten Frequenz (5e4_{II}); und
vi. Spektralbereich-Merkmale (5e7), nämlich:
1. Zeit-Frequenz-Spektrum-Ereignis-Merkmale (5e7_{II});
2. Statistische Merkmale der momentanen dominanten Frequenz (5e7_{III});
vii. Klassifizierung durch ein Machine-Learning-Modell (5e10);
wobei optional in einem Schritt (IV) die Ausgabe der Klassifizierung durch eine Ausgabevorrichtung (6) in haptischer und/oder akustischer und/oder optischer Form erfolgt.

6. Computerimplementiertes Verfahren zur Bearbeitung und Auswertung vibroakustischer Signale gemäß Anspruch 4 zur indirekten Audifikation für die indirekten Prüfung einer Nadelpunktion, umfassend die folgenden Schritte:
(0) Bereitstellen eines digitalisierten vibroakustischen Signals;
(I) Signalvorverarbeitung des bekannten vibroakustischen Signals (5a), umfassend die Prozessschritte:
i. Entfernung kontinuierlicher Komponenten (DC) aus dem VA-Signal (5a1)
ii. Reduzierung des Hintergrundrauschens (5a2)
iii. Signalverbesserung (5a3);
(II) Identifizierung von Segmenten/Regionen von Interesse (5b); und
(III) Indirekte Audifikation (5d2), umfassend die Prozessschritte:
i. Merkmalsextraktion (5d2_{I});
ii. Mapping der extrahierten Merkmale (5d2_{II});
iii. Erzeugung von Klangeigenschaften (5d2_{III}); und
(IV) Übertragung (5d3) an die Ausgabevorrichtung (6).

7. Computerimplementiertes Verfahren zur Bearbeitung und Auswertung vibroakustischer Signale gemäß Anspruch 4 zur automatischen Erkennung von laparoskopischen Instrumenten, umfassend die folgenden Schritte:
(0) Bereitstellen eines digitalisierten vibroakustischen Signals;
(I) Signalvorverarbeitung des bekannten vibroakustischen Signals (5a), umfassend die Prozessschritte:
i. Entfernung kontinuierlicher Komponenten (DC) aus dem VA-Signal (5a1);
ii. Reduzierung des Hintergrundrauschens (5a2);
iii. Signalverbesserung (5a3);
(II) Identifizierung von Segmenten/Regionen von Interesse (5b); und
(III) Klassifizierung (5e) des laparoskopischen Werkzeugs, umfassend die Prozessschritte:
i. Spektralbandzerlegung (5e2);
ii. Identifizierung spektrumbasierter Indikatoren (5e4_{I});
iii. Einspeisen der Daten in einen Deep-Learning-Algorithmus (5e9); und
iv. Klassifizierung durch ein Machine-Learning-Modell (5e10);
wobei optional in einem Schritt (IV) die Speicherung der Klassifizierung in der Ausgabevorrichtung (6) erfolgt.

8. Computerimplementiertes Verfahren zur Bearbeitung und Auswertung vibroakustischer Signale gemäß Anspruch 4 zur direkten Audifikation bei laparoskopischen Eingriffen, umfassend die folgenden Schritte:
(0) Bereitstellen eines digitalisierten vibroakustischen Signals;
(I) Signalvorverarbeitung des bekannten vibroakustischen Signals (5a), umfassend die Prozessschritte:
i. Entfernung kontinuierlicher Komponenten (DC) aus dem VA-Signal (5a1);
ii. Reduzierung des Hintergrundrauschens (5a2);
iii. Signalverbesserung (5a3);
(II) Identifizierung von Segmenten/Regionen von Interesse (5b); und
(III) Direkte Audifikation (5d1), umfassend die Prozessschritte:
i. Filterung des Signals (5d1_{I)});
ii. Anpassung der Lautstärke (5d1_{II});
iii. Bearbeitung der Dynamik (5d1_{III},);
iv. Erzeugung von Effekten (5d1 iv);
(IV) Übertragung (5d3) an die Ausgabevorrichtung (6).

9. Verfahren gemäß einem der Ansprüche 4 bis 8, wobei die vibroakustischen Signale mittels mindestens einer Vorrichtung gemäß einem der Ansprüche 1 bis 3 bereitgestellt werden.

10. Vorrichtung nach einem der Ansprüche 1 bis 3, **dadurch gekennzeichnet, dass** die Vorrichtung eine Daten-/ Signalverarbeitungsvorrichtung (5) umfasst, die vorgesehen ist, das computerimplementierte Verfahren gemäß einem der Ansprüche 4 bis 9 zur Verarbeitung der digitalisierten vibroakustischen Signale auszuführen und das Ergebnis der Verarbeitung bereitzustellen, um eine entsprechend gewünschte Ausgabe auszulösen.
